(19) **Europäisches Patentamt / European Patent Office / Office européen des brevets**

(11) **EP 2 377 542 A2**

(12) **EUROPEAN PATENT APPLICATION**
published in accordance with Art. 153(4) EPC

(43) Date of publication:
19.10.2011 Bulletin 2011/42

(21) Application number: 10731402.3

(22) Date of filing: 15.01.2010

(51) Int Cl.:
*A61K 35/12* (2006.01)    *A61P 19/08* (2006.01)

(86) International application number:
PCT/KR2010/000270

(87) International publication number:
WO 2010/082787 (22.07.2010 Gazette 2010/29)

(84) Designated Contracting States:
AT BE BG CH CY CZ DE DK EE ES FI FR GB GR
HR HU IE IS IT LI LT LU LV MC MK MT NL NO PL
PT RO SE SI SK SM TR

(30) Priority: 15.01.2009  KR 20090003228

(71) Applicant: Corestem Co., Ltd.
Chungcheongbuk-do 363-792 (KR)

(72) Inventors:
• KIM, Kyung Suk
Seoul 135-120 (KR)
• CHOUNG, Jai Jun
Seoul 110-803 (KR)
• YOON, Hyun Soo
Seoul 137-130 (KR)
• LEE, Jun Ho
Seoul 133-817 (KR)
• KU, Sae Kwang
Daegu 706-920 (KR)

(74) Representative: Winkler, Andreas Fritz Ernst
Forrester & Boehmert
Pettenkoferstrasse 20-22
80336 München (DE)

(54) **PHARMACEUTICAL COMPOSITION FOR BONE-DISEASE TREATMENT OR COUNTERING INFLAMMATION, COMPRISING CARTILAGE STEM CELLS AS AN ACTIVE PRINCIPLE**

(57) Disclosed is a pharmaceutical composition for treating bone disease or countering inflammation containing cartilage stem cells as an active ingredient. The present disclosure provides a novel cell treatment regimen for bone disease and inflammatory disease using the cartilage stem cells as an active ingredient. When the cartilage stem cells of the present disclosure are administered into the articular capsule, the administered cartilage stem cells are highly effective in treating bone disease and inflammatory bone disease since they grow or differentiate into chondrocytes and exhibit an anti-inflammatory activity. Since the cartilage stem cells of the present disclosure do not express histocompatibility antigens which give rise to rejection in tissue or organ transplants, autologous cells or exogenous cells can be used during cell transplantation for treatment of bone disease and inflammation.

Fig. 1

EP 2 377 542 A2

## Description

## TECHNICAL FIELD

The present disclosure relates to a pharmaceutical composition for treating bone disease or countering inflammation, containing cartilage stem cells as an active ingredient.

## BACKGROUND

Bones support the soft tissues of the body and body weight, and protect the internal organs from external impact by enclosing them. Also, they mechanically support the muscles and act as reserves of minerals important for the body, most notably calcium, phosphorus and magnesium.

A joint exists between bones. Joints can be classified into immobile joints permitting little or no mobility like in the skull or dental root, movable joints permitting a variety of movements like in the arms and legs or the jaw, and slightly movable, amphiarthrodial joints. In general, the joint refers to the movable joint. The movable joints are classified into ligamentous joints whereby bones are connected only with ligaments and synovial joints. The synovial joint refers to a joint surrounded by a connective tissue capsule (articular capsule). The synovial fluid, which is the lubricating fluid, is secreted in the articular capsule, and the outside of a lot of ligaments are connected to the outside of the articular capsule to support the joint.

Stem cells are "undifferentiated" cells that can differentiate into diverse specialized cell types. Differentiation is the process by which a less specialized cell becomes a more specialized cell type. For a cell to form various tissue cell types including bones, heart, skin, etc., "differentiation" is required. The undifferentiated stem cells can differentiate into various tissue cells under appropriate conditions. Accordingly, mesenchymal stem cells are studied to use their ability to differentiate for regeneration of damaged joint tissue, treatment of inflammation, or the like (US Patent No. 6835377). Also, bone marrow-derived stem cells (Majumdar M. K. et al., J. Cell. Physiol. 185: 98-106, 2000), umbilical cord blood (Gang E. J. et al., Biochem. Biophys. Res. Commun. 321: 102-108, 2004), synovial membrane (Fickert S. et al., Osteoarthritis Cartilage 11: 790-800, 2003), or the like are studied as available cell source for treatment of damaged cartilage in addition to the patient's autologous chondrocytes.

However, the treatment of bone disease or inflammation using stem cells is not so effective at present. Especially, the treatment of bone disease or inflammation using cartilage stem cells has never been reported.

Throughout the specification, a number of publications and patent documents are referred to and cited. The disclosure of the cited publications and patent documents is incorporated herein by reference in its entirety to more clearly describe the state of the related art and the present disclosure.

## DETAILED DESCRIPTION OF THE INVENTION

The inventors of the present disclosure have made efforts to develop stem cells capable of effectively treating bone disease or inflammation without causing immune response in the human body. As a result, they have found out that the cartilage stem cells obtained from the cartilage tissue are very effective in treating bone disease and inflammation.

The present disclosure is directed to providing a pharmaceutical composition for treating bone disease.

The present disclosure is also directed to providing a pharmaceutical composition for countering inflammation.

Other features and aspects will be apparent from the following detailed description, drawings, and claims.

In one general aspect, the present disclosure provides a pharmaceutical composition for treating bone disease, containing: (a) a therapeutically effective amount of cartilage stem cells (i) being immunologically positive for at least one surface antigen selected from a group consisting of CD29, CD44, CD49C, CD73 and CD105, (ii) being immunologically negative for the surface antigen CD34 or CD45, and (iii) expressing at least one undifferentiated stem cell marker protein selected from a group consisting of Oct3/4, Nanog, Rex-1 Sox 2, Sox 5, Sox 6 and Sox 9 in the cytoplasm; and (b) a pharmaceutically acceptable excipient.

In another general aspect, the present disclosure provides a pharmaceutical composition for countering inflammation, containing: (a) a therapeutically effective amount of cartilage stem cells (i) being immunologically positive for at least one surface antigen selected from a group consisting of CD29, CD44, CD49C, CD73 and CD105, (ii) being immunologically negative for the surface antigen CD34 or CD45, and (iii) expressing at least one undifferentiated stem cell marker protein selected from a group consisting of Oct3/4, Nanog, Rex-1, Sox 2, Sox 5, Sox 6 and Sox 9 in the cytoplasm; and (b) a pharmaceutically acceptable excipient.

The inventors of the present disclosure have made efforts to develop stem cells capable of effectively treating bone disease or inflammation without causing immune response in the human body. As a result, they have found out that the cartilage stem cells obtained from the cartilage tissue are very effective in treating bone disease and inflammation.

Stem cells are largely classified into two types: pluripotent stem cells including embryonic stem cells (ES) and embryonic germ cells (EG), and multipotent stem cells. The cartilage stem cells (or chondrogenic stem cells) used in the composition of the present disclosure as an active ingredient are multipotent stem cells derived from the cartilage tissue. Also, the cartilage stem cells of the present disclosure are adult stem cells capable of differentiating into the cartilage tissue and having self-renewal ability.

The cartilage stem cells of the present disclosure are immunologically positive for at least one, specifically at least two, more specifically at least three, further more specifically at least four, most specifically at least five stem cell marker surface antigens selected from a group consisting of CD29, CD44, CD49C, CD73 and CD105. According to a specific embodiment of the present disclosure, the cartilage stem cells of the present disclosure express the surface antigens CD29, CD44, CD49C, CD73 and CD105 80-100%, more specifically 90-100%, most specifically 100%.

And, the cartilage stem cells of the present disclosure are immunologically negative for the hematopoietic stem cell marker surface antigen CD34 and/or CD45. According to a specific embodiment of the present disclosure, the cartilage stem cells of the present disclosure express the hematopoietic stem cell marker surface antigen CD34 and/or CD45 0-10%, more specifically 0.1-5%, most specifically 0.19-1.82%.

The unit "%" used to describe the expression rate of the surface antigen refers to the proportion of the cells wherein the surface antigen is expressed from among the analyzed cells. For instance, if the expression rate of the surface antigen CD29 is 100%, it means that all the cells in the analyzed sample express the surface antigen CD29.

The cartilage stem cells of the present disclosure express at least one, specifically at least two, more specifically at least four, further more specifically at least six, most specifically at least seven undifferentiated stem cell marker proteins selected from a group consisting of Oct3/4, Nanog, Rex-1, Sox 2, Sox 5, Sox 6 and Sox 9.

According to a specific embodiment of the present disclosure, the population doubling time of the cartilage stem cells of the present disclosure is 15-30 hours. More specifically, the population doubling time of the cartilage stem cells of the present disclosure is 17-27 hours, further more specifically 19-25 hours, most specifically 21-23 hours.

According to a specific embodiment of the present disclosure, the cartilage stem cells of the present disclosure do not express the histocompatibility antigen HLA-DR (MHC class 11). Accordingly, the cartilage stem cells of the present disclosure do not cause rejection in tissue or organ transplant, and may treat bone disease or inflammation. Due to these characteristics, not only the autologous cartilage stem cells but also the exogenous cartilage stem cells can be used without rejection.

According to a specific embodiment of the present disclosure, the cartilage stem cells express the cytoskeleton marker protein vimentin or the intercellular cell adhesion protein CD54 (intercellular cell adhesion molecule-1: ICAM-1) in the cytoplasm, and express the intercellular adhesion protein CD44 (homing cell adhesion molecule: HCAM) in the cell membrane.

Specifically, the cartilage stem cells used in the present disclosure may be obtained from the cartilage tissue of mammals, more specifically from human, pig, cow, sheep, rabbit, mouse or rat, further more specifically from human, pig or mouse, most specifically from human.

The cartilage stem cells used in the present disclosure may be obtained by the method described in the Examples section. For example, cartilage tissue is cut and cultured directly to outgrow the cells. Then, the grown cells are subcultured to obtain cartilage stem cells. The medium used for the culturing may be any one commonly used to culture animal cells. For example, Eagle's minimum essential medium (MEM, Eagle, H. Science 130: 432(1959)), $\alpha$-MEM (Stanner, C.P. et al., Nat. New Biol. 230: 52(1971)), Iscove's MEM (Iscove, N. et al., J. Exp. Med. 147: 923(1978)), 199 medium (Morgan et al., Proc. Soc. Exp. Bio. Med., 73: 1 (1950)), CMRL 1066, RPMI 1640 (Moore et al., J. Amer. Med. Assoc. 199: 519(1967)), F12 (Ham, Proc. Natl. Acad. Sci. USA 53: 288(1965)), F10 (Ham, R.G. Exp. Cell Res. 29: 515(1963)), Dulbecco's modification of Eagle's medium (DMEM, Dulbecco, R. et al., Virology 8: 396(1959)), DMEM/F12 mixture (Barnes, D. et al., Anal. Biochem. 102: 255(1980)), Waymouth's MB752/1 (Waymouth, C. J. Natl. Cancer Inst. 22: 1003(1959)), McCoy's 5A (McCoy, T.A., et al., Proc. Soc. Exp. Biol. Med. 100: 115(1959)), MCDB series (Ham, R.G. et al., In Vitro 14: 11 (1978)), or the like may be used. Specifically, the medium used for the culturing may further contain serum (e.g., fetal bovine serum), alanine and glutamine.

After subculturing to 70-80% confluency, the adherent cells are harvested and some of them are used for the next subculturing. Through this process, the cartilage stem cells capable of differentiating into chondrocytes and having stem cell characteristics are established.

According to a specific embodiment of the present disclosure, the cartilage stem cells of the present disclosure are able to grow or differentiate into chondrocytes. That is to say, the cartilage stem cells of the present disclosure can grow or differentiate into chondrocytes at the site where the cartilage stem cells were injected, specifically in the articular capsule, and thus exhibit therapeutic effect for various bone disease, caused specifically by bone damage, more specifically by defects of the cartilage tissue.

According to a specific embodiment of the present disclosure, the cartilage stem cells of the present disclosure exhibit an anti-inflammatory activity (see Example 4, Table 8). To the best knowledge of the inventors, such anti-inflammatory activity of the cartilage stem cells is first demonstrated and presented in the present disclosure. As described in the Examples section, the cartilage stem cells of the present disclosure exhibit better anti-inflammatory activity than the existing anti-inflammatory drug diclofenac. The superior anti-inflammatory activity of the cartilage stem cells is very interesting and may lead to development of new cell therapy for bone disease or inflammation.

According to the most specific embodiment of the present disclosure, the human cartilage stem cells used in the present disclosure may be those under the accession numbers KCTC 11397BP "CBAC266", KCTC 11617BP "CBAC237", KCTC 11618BP "CBAC240" or KCTC 11619BP "CBAC1014".

The bone disease that may be treated by the pharmaceutical composition of the present disclosure is a bone disease caused by pathological or physical damaged to the bone or cartilage tissue. Specifically, it may be articular cartilage damage (e.g., meniscus damage, herniated disk), osteoarthritis, osteoporosis, osteomalacia, rickets, osteitis fibrosa, aplastic bone disease, metabolic bone disease, osteolysis, leukopenia, bone deformity, hypercalcemia, nerve compression syndrome or physical bone damage, most specifically osteoarthritis.

As used herein, the term "therapeutically effective amount" refers to an amount sufficient to achieve the therapeutic effect or activity of the cartilage stem cells described above.

When the composition of the present disclosure is prepared as a pharmaceutical composition, the pharmaceutical composition of the present disclosure may comprise a pharmaceutically acceptable excipient. The pharmaceutically acceptable excipient included in the pharmaceutical composition of the present disclosure is one commonly used in the preparation of formulations and includes lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, physiological saline, phosphate buffered saline (PBS) or culture medium, but is not limited thereto.

The pharmaceutical composition of the present disclosure may further include, in addition to above-described components, a lubricant, a wetting agent, a sweetener, a fragrance, an emulsifier, a suspending agent, a preservative, or the like. Suitable pharmaceutically acceptable excipients and formulations are described in detail in Remington's Pharmaceutical Sciences (19th ed., 1995).

The pharmaceutical composition of the present disclosure may be administered orally or parenterally, specifically parenterally, most specifically intraarticularly.

An appropriate dosage of the pharmaceutical composition of the present disclosure may be determined variously depending on such factors as preparation method, administration method, age, body weight and sex of the patient, pathological condition, diet, administration time, administration route, excretion rate or response sensitivity. A general dosage of the pharmaceutical composition of the present disclosure for an adult is $10^2$-$10^{10}$ cells per day.

The pharmaceutical composition of the present disclosure may be prepared into a unit dosage form or multiple dosage form along with a pharmaceutically acceptable excipient and/or carrier according to a method that can be easily employed by those skilled in the art. The formulation may be in the form of solution in oily or aqueous medium, suspension, syrup, emulsion, extract, dust, powder, granule, tablet or capsule, and may further include a dispersant or stabilizer.

The inflammatory disease that may be treated by the pharmaceutical composition for countering inflammation of the present disclosure includes all the diseases caused by inflammatory response in the cartilage tissue. Specifically, the inflammatory disease that may be treated by the pharmaceutical composition for countering inflammation of the present disclosure is an inflammatory arthritis, more specifically osteoarthritis, rheumatoid arthritis, ankylosing spondylitis, psoriatic arthritis, juvenile rheumatoid arthritis, reactive arthritis (Reiter's syndrome) or enteropathic arthritis, most specifically osteoarthritis or rheumatoid arthritis.

According to a specific embodiment of the present disclosure, the pharmaceutical composition for countering inflammation of the present disclosure is a composition for intraarticular administration. When the composition is administered into the articular capsule, the cartilage stem cells provide therapeutic effect for inflammatory arthritis by exhibiting anti-inflammatory activity in the articular capsule.

The features and advantages of the present disclosure may be summarized as follows:

(i) The present disclosure provides a novel cell treatment regimen for bone disease using cartilage stem cells as an active ingredient.
(ii) Also, the present disclosure provides a novel cell treatment regimen for inflammatory disease using cartilage stem cells as an active ingredient.
(iii) The cartilage stem cells of the present disclosure are very effective in treating bone disease and inflammatory disease since they promote growth of chondrocytes and exhibit anti-inflammatory activity when they are administered into the articular capsule.
(iv) To the best knowledge of the inventors, the anti-inflammatory activity of the cartilage stem cells is first demonstrated and presented in the present disclosure.
(v) The cartilage stem cells of the present disclosure provide better anti-inflammatory activity than the existing anti-inflammatory drug diclofenac.
(vi) Since the cartilage stem cells of the present disclosure do not express histocompatibility antigens which give rise to rejection in tissue or organ transplants, autologous cells or exogenous cells can be used during cell transplantation for treatment of bone disease and inflammation.

**BRIEF DESCRIPTION OF THE DRAWINGS**

FIGS. 1a-1d respectively show images of cartilage-derived stem cells grown from human articular cartilage tissue on a cell culture dish (FIG. 1a: CBAC266, FIG. 1b: CBAC237, FIG. 1c: CBAC240, FIG. 1d: CBAC1014).

FIGS. 2a-2d respectively show images of cartilage-derived stem cells at passage 8. It can be seen that the cells maintain their shape (FIG. 2a: CBAC266, FIG. 2b: CBAC237, FIG. 2c: CBAC240, FIG. 2d: CBAC1014).

FIGS. 3a-3d show growth rate of human cartilage stem cells with culture period (FIG. 3a: CBAC266, FIG. 3b: CBAC237, FIG. 3c: CBAC240, FIG. 3d: CBAC1014).

FIGS. 4a-4d show a result of analyzing surface antigens of human cartilage stem cells at passage 15 by FACS (FIG. 4a: CBAC266, FIG. 4b: CBAC237, FIG. 4c: CBAC240 , FIG. 4d: CBAC1014).

FIG. 5 shows an image of chromosomes of human cartilage-derived stem cells CBAC266 (passage 15).

FIG. 6 shows fine structure of human cartilage-derived stem cells CBAC266 (passage 15).

FIG. 7 shows immunohistochemically stained human cartilage-derived stem cells CBAC266 (passage 15).

FIG. 8 shows human cartilage-derived stem cells CBAC266 cultured into pellet for 4 weeks in a cartilage differentiation medium.

FIGS. 9a-9e show fine structure of human cartilage-derived stem cells CBAC266 cultured into pellet for 4 weeks.

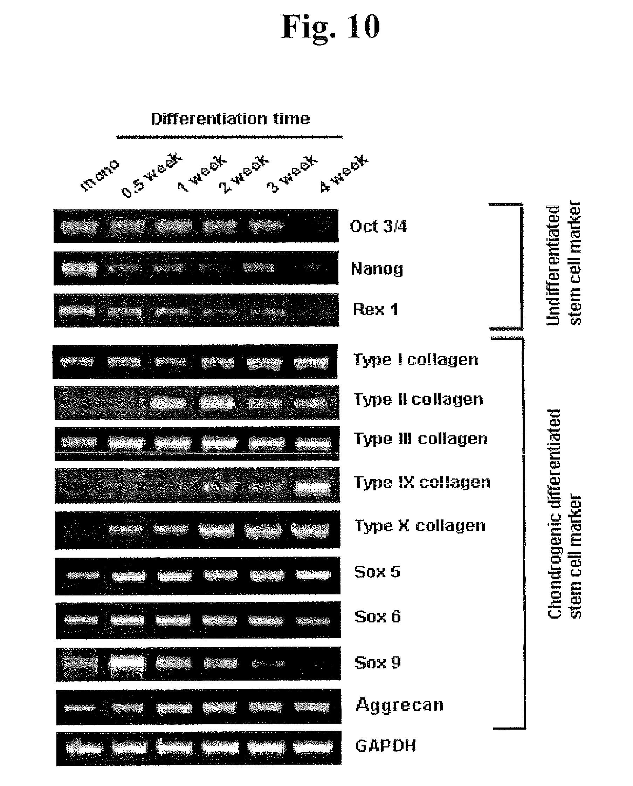

FIG. 10 shows a result of analyzing marker genes of human cartilage-derived stem cells CBAC266 cultured into pellet for 4 weeks.

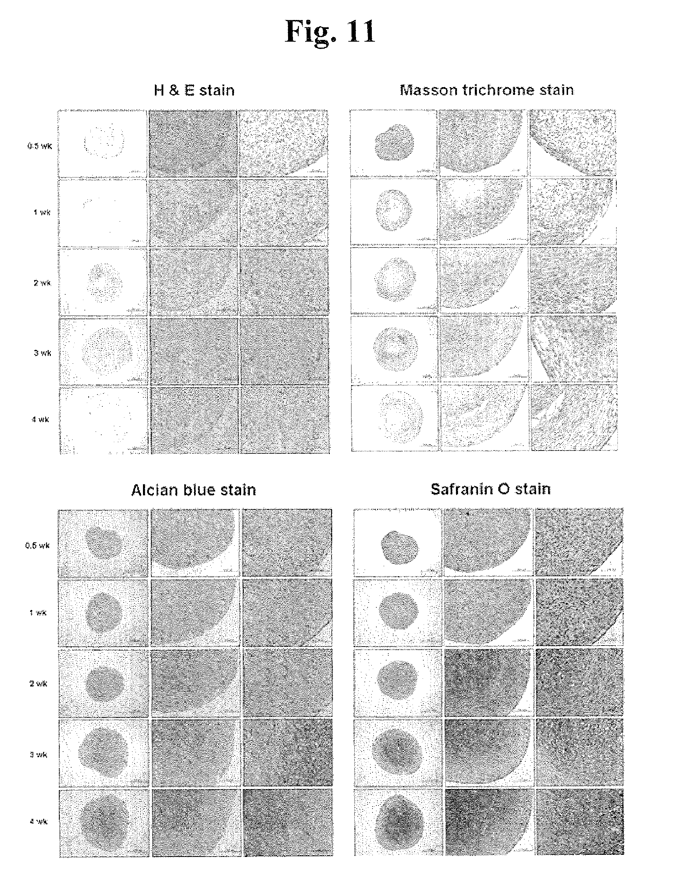

FIG. 11 shows immunohistochemically stained human cartilage-derived stem cells CBAC266 cultured into pellet for 4 weeks.

FIG. 12 shows a result of analyzing change in knee joint thickness after injecting human cartilage-derived stem cells CBAC266 to the knee joint.

FIG. 13 shows a result of analyzing change in maximum knee joint angle after injecting human cartilage-derived stem cells CBAC266 to the knee joint.

FIG. 14 shows histopathologically stained cartilage surface of the femur and the after injecting human cartilage-derived stem cells CBAC266 to the femur and the tibia.

FIG. 15 shows BrdU-positive cells on the cartilage surface of the femur and the after injecting human cartilage-derived stem cells CBAC266 to the femur and the tibia.

FIG. 16 shows immunohistochemically stained cartilage after injecting human cartilage-derived stem cells CBAC266 to the cartilage.

The examples and experiments will now be described. The following examples and experiments are for illustrative purposes only and not intended to limit the scope of this disclosure.

**EXAMPLES**

**Example 1: Isolation and culturing of cartilage-derived stem cells**

*Collection of tissue*

Four cartilage tissues were collected from the cartilage of human embryo in the stage of ossification. For transport of the cartilage tissue samples, a sterilized plastic container holding Dulbecco's phosphate-buffered saline (DPBS; Gibco, USA) containing 5 IU/mL heparin (Hanlim Pharm., Korea) and 1% penicillin-streptomycin (Gibco, USA) was used to maintain aseptic condition. The tissue samples were washed at least 3 times with DPBS containing 1% penicillin-streptomycin (Gibco, USA) to remove residual blood and then transferred to 100 Ø Petri dishes holding DPBS.

*Isolation and culturing of cartilage-derived stem cells*

Four cartilage tissues were isolated using a dissecting microscope (Olympus, USA) from the four tissue samples transferred to the Petri dishes holding the washing medium. The isolated cartilage tissues were respectively transferred to sterile 50-mL tubes (Falcon, USA) and cut to small pieces (< 0.5 $_{CM}{}^2$) using a sterile scalpel. After adding a cell culture medium and resuspending by pipetting, cells were cultured in a cell culture dish containing medium under the condition of 37°C and 7% $CO_2$. The cells grown from the tissues could be observed 3-4 days later with an optical microscope (Nikon, Japan). The cell culture medium was replaced every 3 or 4 days.

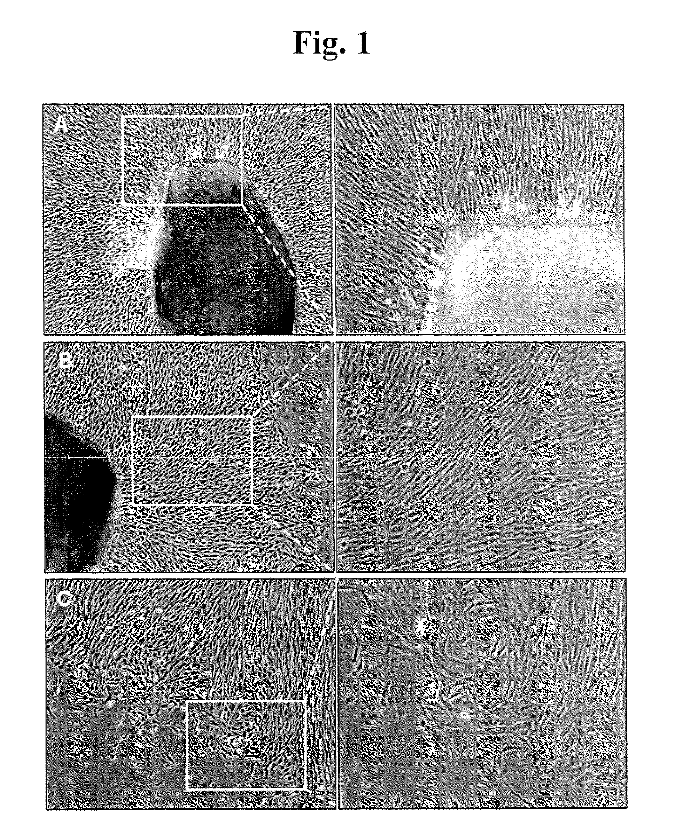

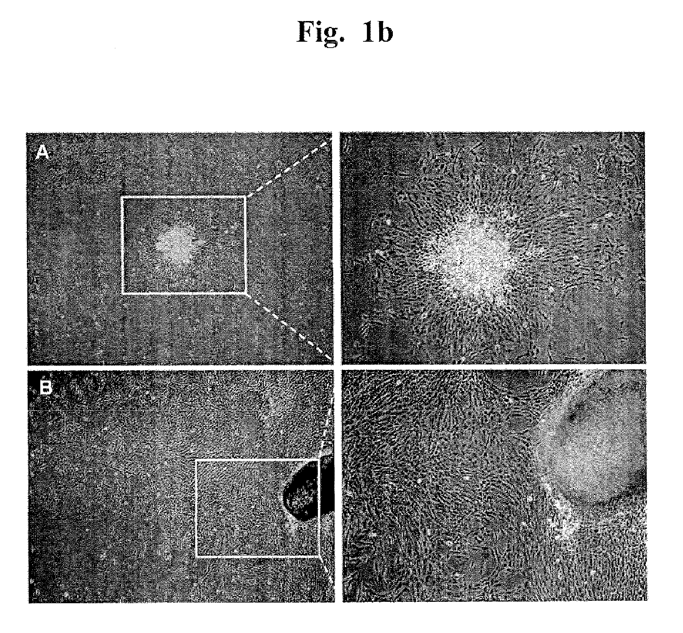

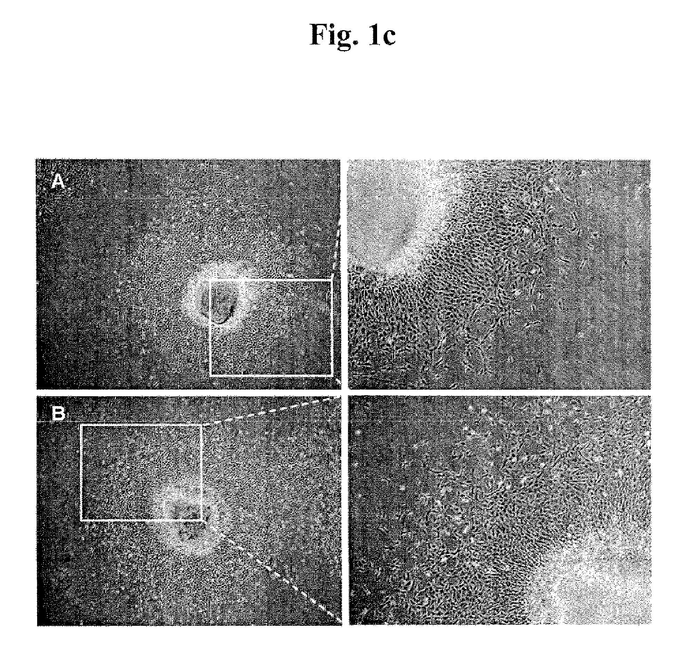

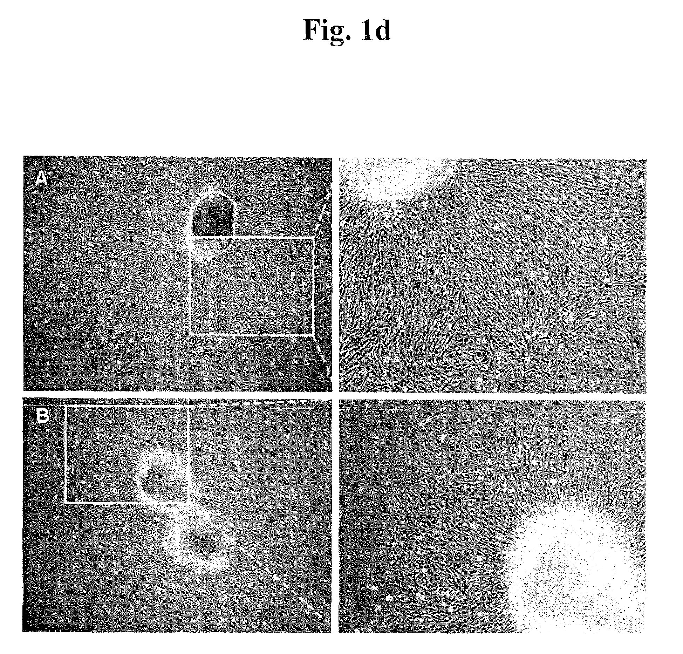

The cells grown in the 60-mm cell culture dish (Nunc, USA) for 11 days proliferate 70-80% or more. All the grown single cells show polygonal shapes and show uniform growth pattern of spindle form typical of the dendritic fibroblasts (FIGS. 1a-1d).

CoreStem Basal Medium (CSBM; Corestem Inc., Korea) was used as the basal medium. CSBM containing 1% penicillin-streptomycin was used as the washing medium, and 10% (v/v) fetal bovine serum (FBS; HyClone, USA) was added to use as the cell culture medium.

For subculturing, the adherent cells that adhered to the bottom of the cell culture dish (Nunc, USA) 70-80% or more were harvested by treating with trypsin (0.125% trypsin-EDTA). Counting and viability measurement of the harvested cells were performed by the Trypan blue dye exclusion method. The harvested cells were determined as passage 0 (P0).

Among the human cartilage stem cell lines established by the above process, one cartilage stem cell line was named as "CBAC266" and deposited in the Korean Collection for Type Cultures (KCTC) on October 2, 2008 under the accession number KCTC 11397BP.

Also, among the human cartilage stem cell lines established through the process, three cartilage stem cell lines was named as "CBAC237", "CBAC240" and "CBAC1014", respectively, and deposited in the KCTC on January 8, 2010 under the accession numbers KCTC 11617BP, KCTC 11618BP and KCTC 11619BP, respectively.

*Cryopreservation of cartilage-derived stem cells*

The four cartilage-derived stem cells were inoculated to a T175 cell culture flask (Nunc, USA) with $1.5 \times 10^5$ to $2 \times 10^5$ cells per flask and cultured under the condition of 37 °C and 7% $CO_2$ for 7 days. During the culturing, the medium was replaced every 3 or 4 days.

The cells grown 70-80% or more were harvested by treating with trypsin (0.125% trypsin-EDTA). The harvested cells were suspended in 500 μL of CSBM containing 20% fetal bovine serum and 10% dimethyl sulfoxide (DMSO; Sigma, USA) as cryopreservation medium so that the density of the cells were $6 \times 10^5$ cells/vial. Then, the cells were transferred to a cryotube vial (Nunc, USA).

The cell suspensions held in the vials were put in cryopreservation containers (Nalgene, USA) containing isopropyl alcohol and stored in an ultra-low temperature freezer at -80°C for 24 hours. The next day, the vials holding the cells were transferred to and stored in a liquid nitrogen tank.

*Thawing of cartilage-derived stem cells*

Each vial containing the cells at $6 \times 10^5$ cells/vial was transferred from the liquid nitrogen tank to room temperature. After removing liquid nitrogen, the vial was quickly transferred to a water bath at 37°C. The four thawed cell suspensions held in the vials were inoculated to cell culture flasks containing cell culture medium in sterilized state.

The density of the inoculated cells was $1.5 \times 10^5$ to $2 \times 10^5$ cells per flask, respectively.

The cell culture medium was replaced every 3 or 4 days. The shape and growth of the cells were observed an optical microscope (Nikon, Japan).

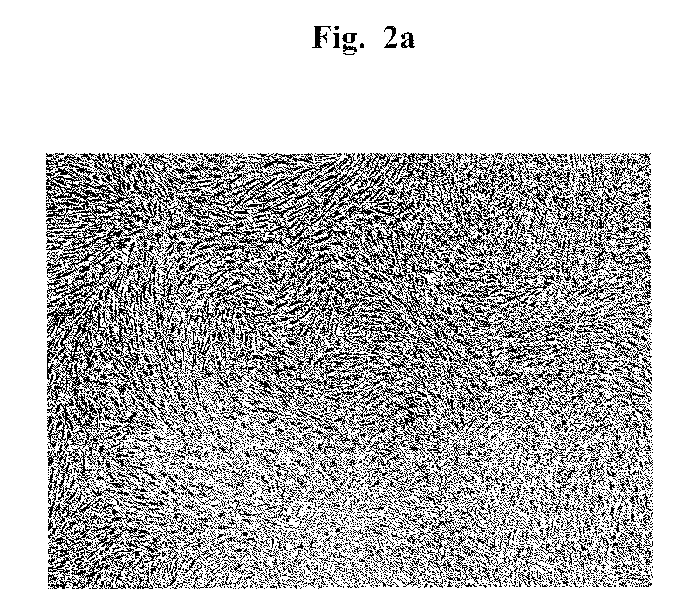

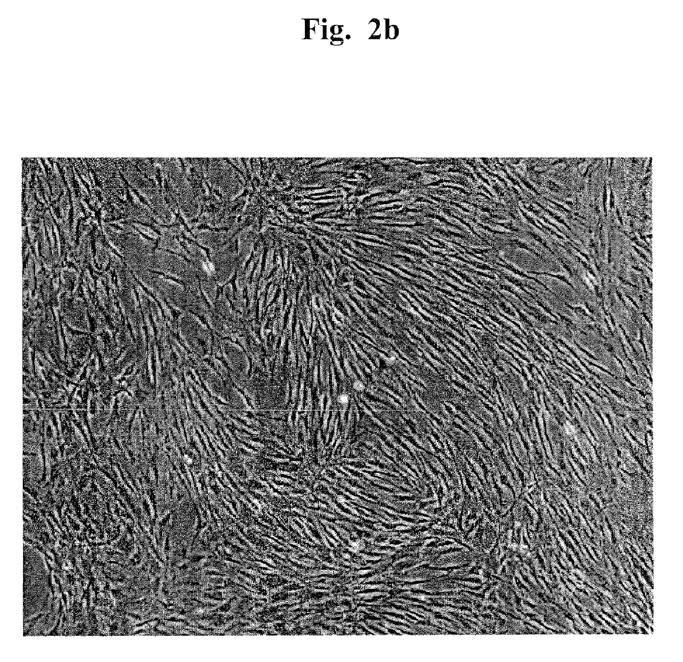

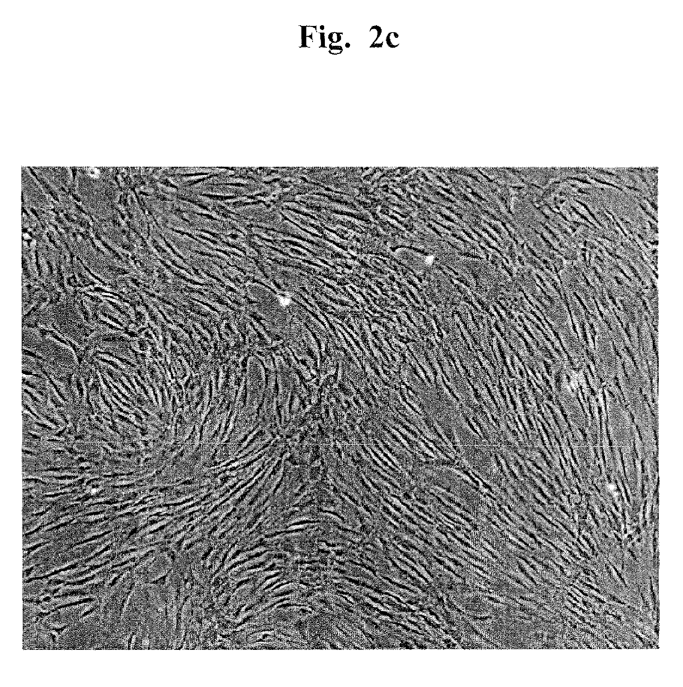

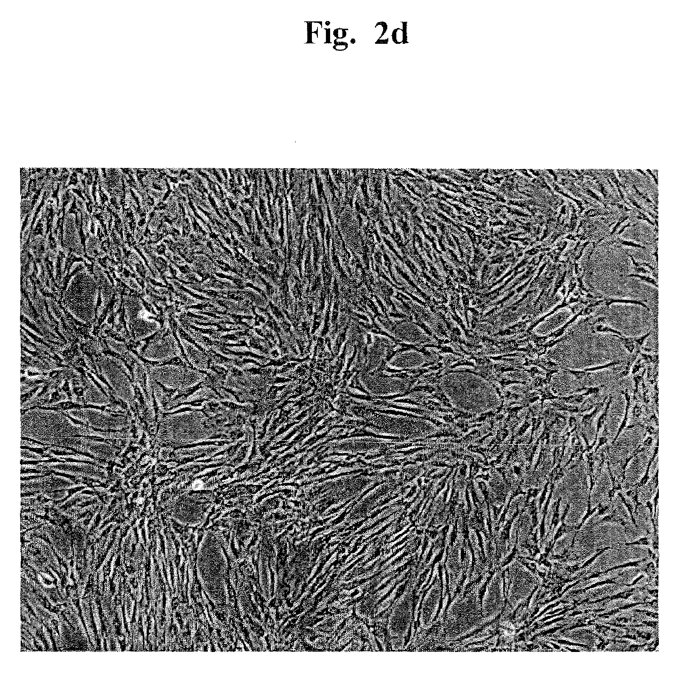

The cellular shape was observed at passage 8 (P8). As shown in FIGS. 1a-1d, the cells at passage 0 (P0) showed uniform growth pattern of spindle form typical of the dendritic fibroblasts (FIGS. 2a-2d).

**Example 2: Characterization of cartilage-derived stem cells**

*Growth rate of cartilage stem cells*

The growth rate of the four isolated cartilage-derived stem cells was measured with increasing the passage number.

Population doubling (PD) is an index indicating the cell growth rate. The population doubling (PD) can be calculated from the equation: PD = log (number of finally harvested cells / number of initially inoculated cells) / log 2. The number of the initially inoculated cells was $1.5 \times 10^5$. The number of the cells harvested after the culturing was counted to determine PD.

The four cartilage-derived stem cells isolated from the cartilage tissue as in Example 1 were subcultured.

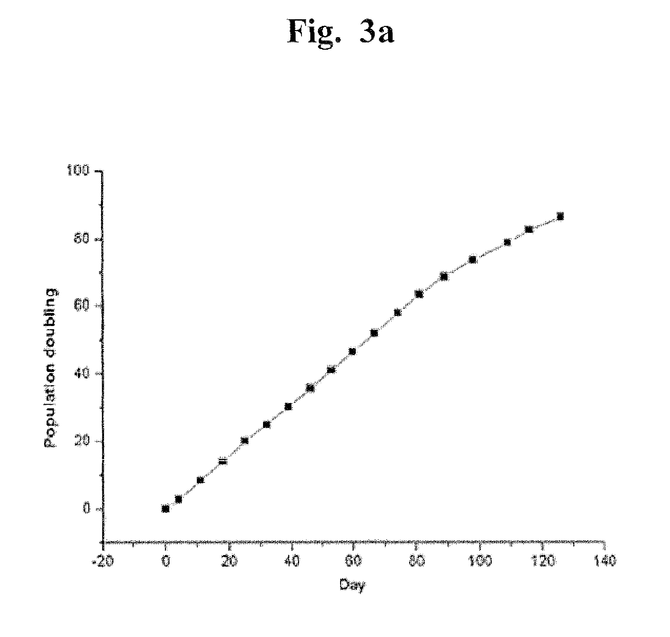

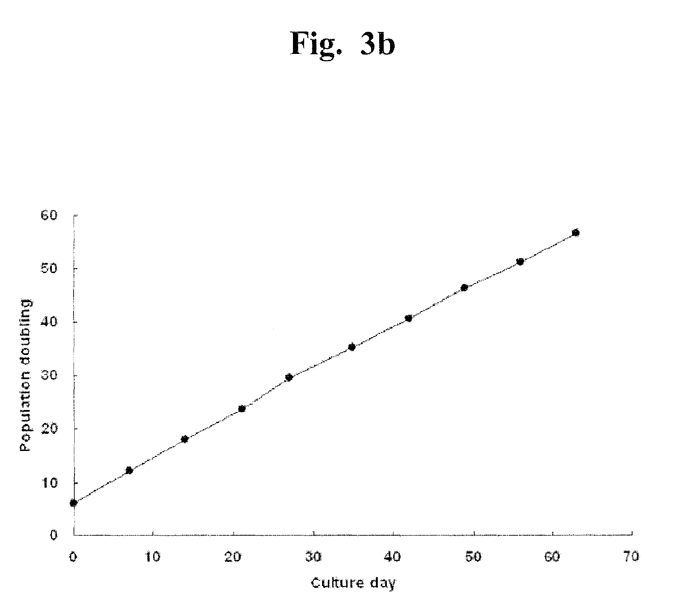

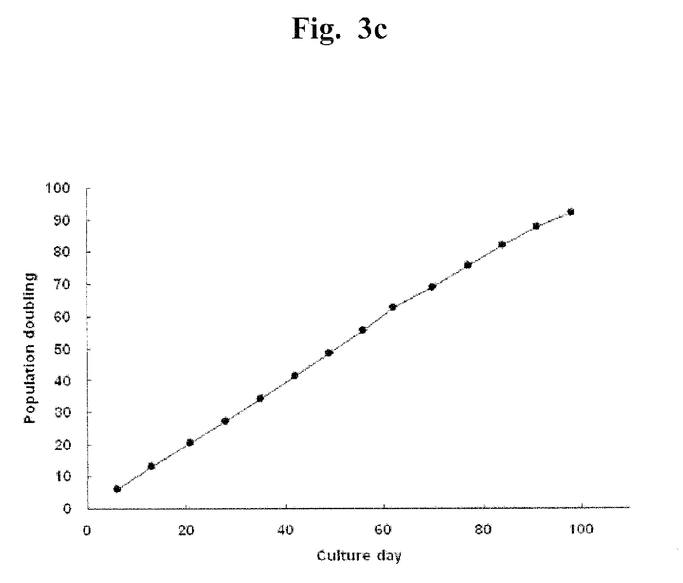

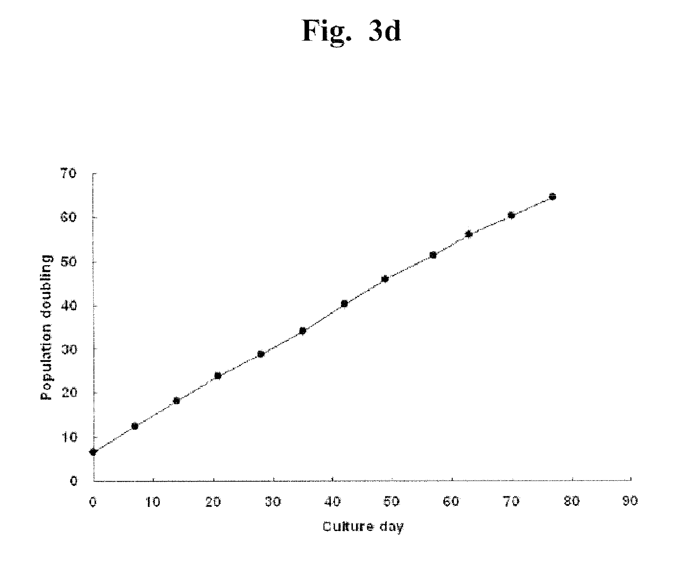

After 130 days (P8-P23), the population doubling time (PDT) was $22 \pm 3$ hours for the CBAC226 cells, $30 \pm 2.8$ hours for the CBAC237 cells, $25.9 \pm 4.2$ hours for the CBAC240 cells, and $32.2 \pm 4.1$ hours for the CBAC1014 cells (FIGS. 3a-3d).

*Expression of surface antigens of cartilage- derived stem cells*

The expression of the stem cell marker surface antigens of the isolated cartilage-derived stem cells was analyzed by fluorescence-activated cell sorting (FACS). The four stem cells isolated from the cartilage tissue were respectively inoculated to T75 flasks at $0.7 \times 10^5$ cells per flask and cultured for 7 days under the condition of 37 °C and 7% $CO_2$. The cell culture medium was replaced every 3 or 4 days. The four stem cells grown 70-80% or more were harvested by treating with trypsin (0.125% trypsin-EDTA). The four harvested cells were respectively washed 3 times with DPBS (Gibco, USA) containing 2% fetal bovine serum, suspended at a concentration of $1 \times 10^5$ cells/100 μL, inoculated to test tubes, and analyzed with FACSCalibur (Becton Dickinson, NJ, USA). Mesenchymal stem cell markers PE-CD29, PE-CD49C, PE-CD44, PE-CD73 and PE-CD105, hematopoietic cell markers PE-CD34 and PE-CD45, and histocompatibility antigen marker PE-HLA-DR (MHC class II) were used as antibodies. PE-immunoglobulin isotype IgG1 was used as negative control antibody. All antibodies were acquired from Becton Dickinson (USA). The antibodies were reacted with the cells on ice for 30 minutes in the dark. Flow cytometry was performed after flushing out residual antibodies with DPBS (Gibco, USA).

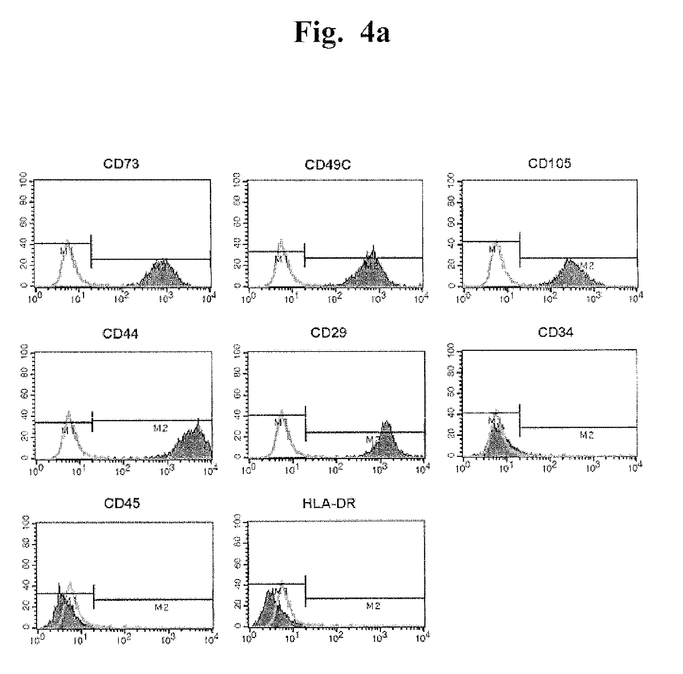

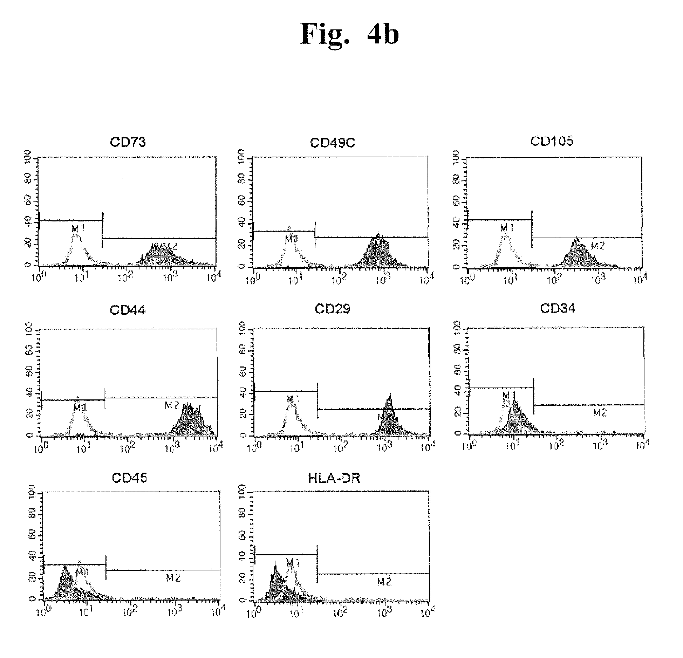

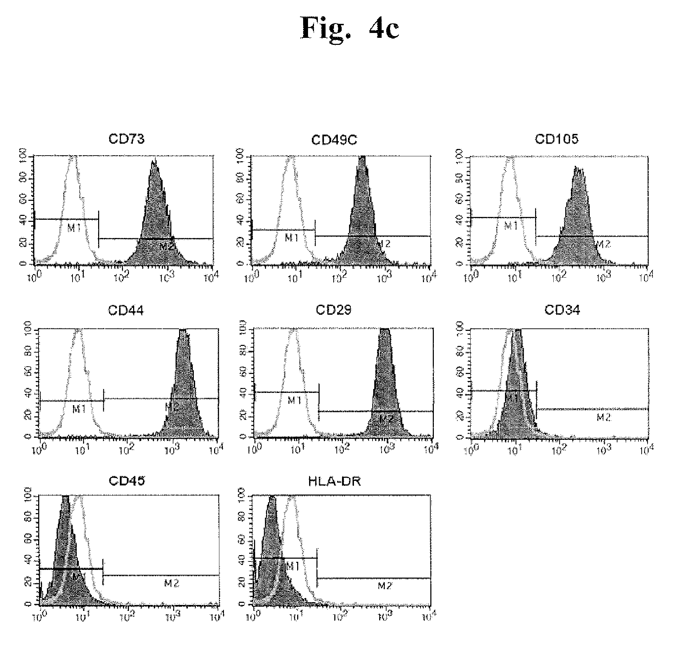

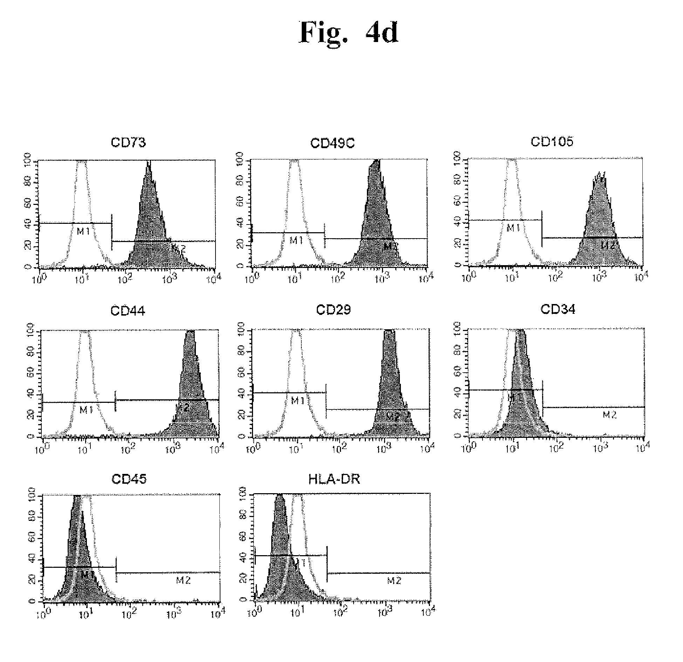

Among the four established cartilage-derived stem cells, at least 95% of the cells were immunologically positive for the stem cell marker surface antigens CD29, CD44, CD49C, CD73 and CD105 and were immunologically negative for the hematopoietic stem cell marker surface antigens CD34 and CD45. Consequently, it can be seen that the four isolated cartilage-derived stem cells have the characteristics of stem cells. Also, since they do not or hardly express the histocompatibility antigen HLA-DR (MHC class II) which gives rise to rejection in tissue or organ transplants, rejection can be avoided during cell transplantation using exogenous cells. Therefore, the four established cartilage-derived stem cells can be used as exogenous cells (see FIGS. 4a-4d and Tables 1-4).

Table 1

| | | | | | | | | |
|---|---|---|---|---|---|---|---|---|
| Cell surface antigens expressed in CBAC226 cartilage stem cells | | | | | | | | |
| Surface antigen | CD29+ | CD49C+ | CD44+ | CD73+ | C0105+ | CD34+ | CD45+ | HLA-DR+ |
| Expression rate (%) | 100.00 | 100.00 | 100.00 | 100.00 | 100.00 | 1.82 | 0.19 | 0.00 |

Table 2

| | | | | | | | | |
|---|---|---|---|---|---|---|---|---|
| Cell surface antigens expressed in CBAC237 cartilage stem cells | | | | | | | | |
| Surface antigen | CD29+ | CD49C+ | CD44+ | CD73+ | CD105+ | CD34+ | CD45+ | HLA-DR+ |
| Expression rate (%) | 100.00 | 99.93 | 99.86 | 99.91 | 99.95 | 2.48 | 0.05 | 0.05 |

Table 3

| | | | | | | | | |
|---|---|---|---|---|---|---|---|---|
| Cell surface antigens expressed in CBAC240 cartilage stem cells | | | | | | | | |
| Surface antigen | CD29+ | CD49C+ | CD44+ | CD73+ | CD105+ | CD34+ | CD45+ | HLA-DR+ |
| Expression rate (%) | 99.98 | 99.72 | 99.89 | 99.92 | 99.80 | 1.47 | 0.12 | 0.04 |

Table 4

| | | | | | | | | |
|---|---|---|---|---|---|---|---|---|
| Cell surface antigens expressed in CBAC1014 cartilage stem cells | | | | | | | | |
| Surface antigen | CD29+ | CD49C+ | CD44+ | CD73+ | CD105+ | CD34+ | CD45+ | HLA-DR+ |
| Expression rate (%) | 99.97 | 99.87 | 99.69 | 99.89 | 99.98 | 1.91 | 0.16 | 0.05 |

*Karyotype analysis of cartilage-derived stem cells*

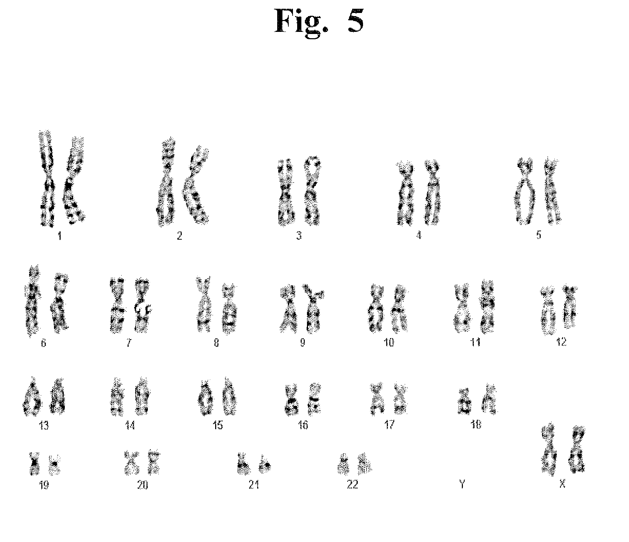

Karyotype analysis was performed to investigate whether the number and appearance of chromosomes are maintained during the Isolation and culturing of the cells. The isolated cells were inoculated to a T25 flask with $1.0-1.2 \times 10^5$ cells per flask and cultured for 2-3 days under the condition of 37 °C and 7% $CO_2$. The grown cells were harvested by treating with trypsin (0.125% trypsin-EDTA), and 25 metaphases of the harvested cells were analyzed by GTG banding. As a result, no abnormality was found in the number or structure of the chromosomes. Therefore, it can be seen that the chromosomes (46 XX) are maintained stably without change in karyotype during the culturing of the cartilage stem cells (FIG. 5).

*Safety test of cartilage-derived stem cells*

It was investigated whether the cells were adventitiously infected by exogenous microorganisms or viruses during the isolation and culturing of the cells. The isolated cells were inoculated to a T75 flask with $0.7 \times 10^5$ cells per flask and cultured for 7 days under the condition of 37°C and 7% $CO_2$, The medium was replaced every 3 or 4 days during the cell culturing. The cells grown 70-80% or ore were harvested by treating with trypsin (0.125% trypsin-EDTA). No exogenous microorganisms or viruses were detected from the harvested cartilage stem cells (Table 5).

Table 5

| Safety test of cartilage-derived stem cells | | |
|---|---|---|
| Safety test | | Result |
| Exogenous microorganisms | Gram staining | negative |
| | Culture & sensitivity | negative |
| Exogenous viruses | HBV DNA | negative |
| | HCV DNA | negative |
| | CMV DNA | negative |
| | HIV | negative |
| | Parvovirus B19 | negative |

*Fine structure analysis of cartilage-derived stem cells*

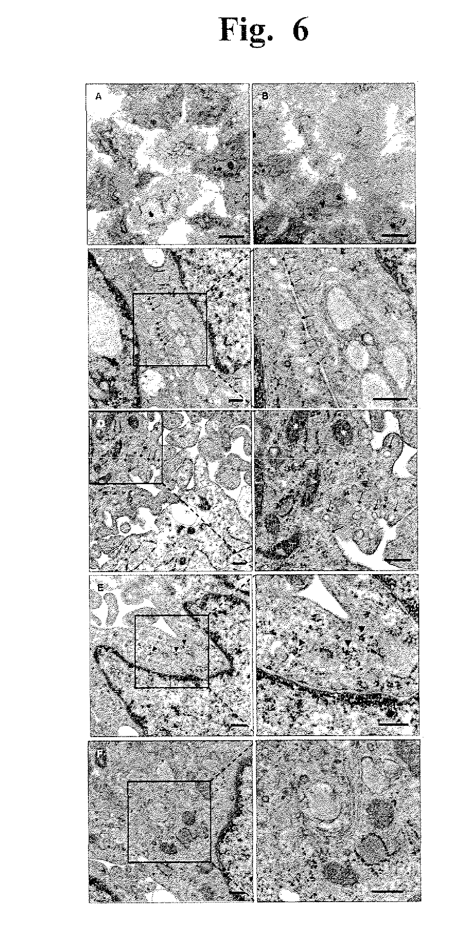

The cells isolated from the cartilage tissue were observed under an electron microscope in order to investigate the morphological feature of fine structure. The isolated cells were inoculated to a T25 flask with $1.0-1.2 \times 10^5$ cells per flask and cultured for 7 days under the condition of 37 °C and 7% $CO_2$. The cell culture medium was replaced every 3 or 4 days. The grown cells were harvested by treating with trypsin, and the harvested cells were washed 3 times with DPBS, fixed in 2.5% glutaraldehyde-2% paraformaldehyde (4 °C, 0.1 M phosphate buffer, pH 7.4) for 2 hours, washed 3 times with PBS (4 °C, 0.1 M phosphate buffer, pH 7.4), and then fixed in 1 % $O_SO_4$ (4 °C, 0.1 M phosphate buffer, pH 7.4) for 1 hour in the dark. The fixed cells were washed 3 times with the same phosphate buffer, dehydrated with ethanol with gradually increasing concentration, tread with propylene oxide and embedded in Epon 812 mixture, and then thermally treated at 60 °C for 48. The resulting block was prepared into 1-$\mu$m thick sections using an ultramicrotome (Ultracut UCT, Leica, Germany), and observed under an optical microscope after staining with toluidine blue. Also, 60-nm thick microsections were doubly stained with uranyl acetate and lead citrate and then observed under a transmission electronic microscope (TEM; H-7600; Hitachi, Japan) at an acceleration voltage of 80 kV. As a result, the cells showed well-developed cytoplasmic processes and microvilli (FIG. 6, A and B), and a number of well-developed secretory granules were observed in the cytoplasm (FIG. 6, C and D). Also, free ribosomes were observed in the cytoplasm and fibrous laminas were observed between the swollen nuclei and the inner nuclear membranes (FIG. 6, E). Further, mitochondria and rough endoplasmic reticula were observed near the nucleus (FIG. 6, F). To conclude, the stem cells showed a fine structure characteristic of the cartilage stem cells.

*Immunohistochemical analysis of cartilage-derived stem cells*

The stem cell marker protein expression pattern of the cells was analyzed by immunofluorescence detection. After inoculating the cells to a 4-well cell culture plate (Nunc, USA) equipped with a glass coverslip pretreated with 0.1% gelatin ($0.5 \times 10^4$ cells/well) and culturing for 7 days under the condition of 37 °C and 7% $CO_2$, the cells grown 70-80% or more were washed 3 times with DPBS and fixed for 30 minutes with 10% neutral-buffered formalin (NBF). The fixed cells were treated for 5 minutes with 0.1% Triton X-100, washed 3 times with DPBS, and blocked for 60 minutes with 3% bovine serum albumin (BSA; Sigma, USA). After the blocking, the cells were washed 3 times with DPBS and reacted for 3 hours respectively with the primary antibodies anti-mouse Oct3/4 (1:25, Santa Cruz, USA), anti-goat Nanog (1:25, Santa Cruz, USA), anti-rabbit Sox2 (1:100, Chemicon, USA), anti-mouse vWF (1:100, Novocastra Lab. Ltd., England), anti-mouse HCAM (CD44, Novocastra Lab. Ltd., England), anti-mouse ICAM (CD54, 1:200, Novocastra Lab. Ltd., England) and anti-mouse vimentin (1:200, Novocastra Lab. Ltd., England), washed 3 times with DPBS, and then reacted for 3 hours with FITC-conjugated anti-rabbit (1:500, Invitrogen, USA) and Cy3-conjugated anti-goat (1:500, Invitrogen. USA) secondary antibodies. Then, after reacting for 10 minutes with the nuclear marker DAPI (1:1000, Sigma, USA), they were washed 3 times with DPBS and protein expression was analyzed by confocal microscopy (LSM 510; Zeiss, Germany).

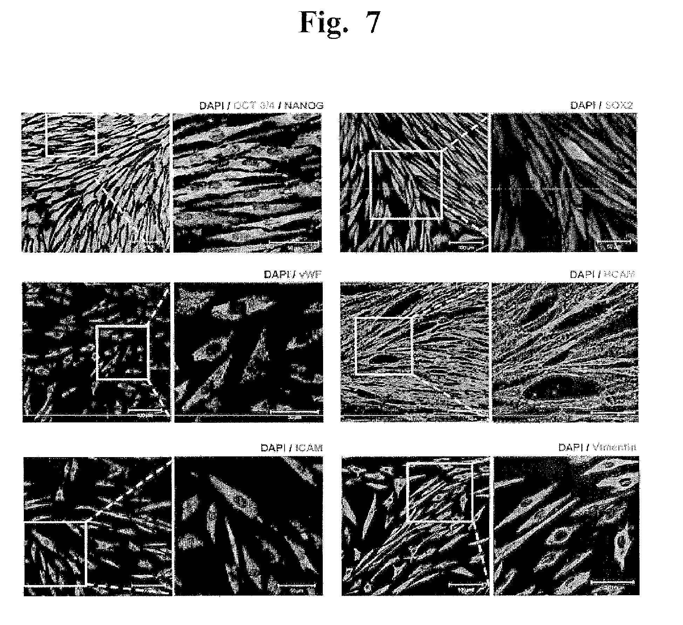

The undifferentiated stem cell marker proteins Oct3/4, Nanog and Sox2, the endothelial cell marker protein von Willebrand Factor (vWF), the cytoskeleton marker protein vimentin and the intercellular adhesion protein CD54 (intercellular cell adhesion molecule-1, ICAM-1) were strongly expressed in the cytoplasm, and the intercellular adhesion protein CD44 (homing cell adhesion molecule, HCAM) was strongly expressed in the cell membrane (FIG. 7).

**Example 3: Characterization of differentiation of cartilage-derived stem cells**

*Inducement of differentiation into chondrocytes.*

After adding a chondrocyte differentiation medium (NH ChondroDiff; Miltenyi Biotec. USA) and culturing the cells into pellets, the morphological characteristics were analyzed.

The cells isolated from the cartilage tissue were inoculated to a T25 flask with $1.0$-$1.2 \times 10^5$ per well and cultured for 7 days under the condition of 37 ˚C and 7% $CO_2$. The grown cells were harvested by treating with trypsin. The harvested cells were suspended in a cell culture medium to a concentration of $5 \times 10^5$ cells/mL and transferred to 15-mL polypropylene conical tubes, 1 mL each. Then, after centrifuging at 500 x g for 5 minutes and removing the supernatant, 1 mL of a chondrocyte differentiation medium (NH ChondroDiff medium; Miltenyi Biotec. USA) was added per each.

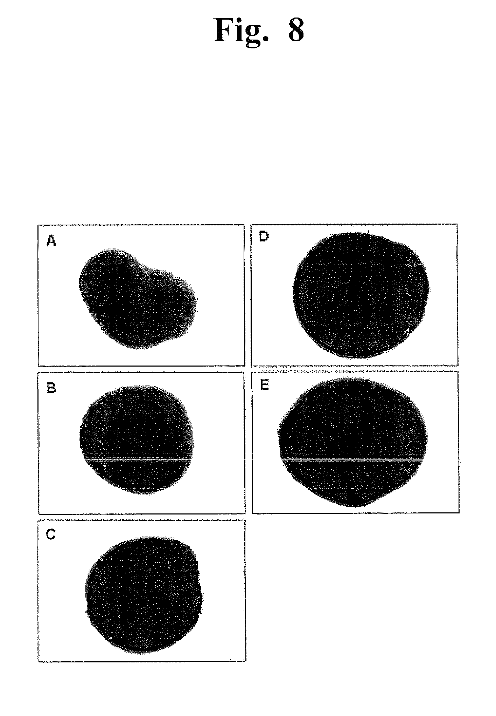

After slightly uncapping the tube to allow circulation of air, the cells were cultured for 0.5, 1, 2, 3 and 4 weeks under the condition of 37 ˚C and 7% $CO_2$. The shape of the cells was observed under an optical microscope (Nikon, Japan). Irregular cell pellets were formed at 0.5 week (FIG. 8, A). The cells grown for 1 to 4 weeks formed spherical pellets. The size of the pellets increased with the culture period (FIG. 8, B, C, D and E).

*Fine structure analysis of differentiated cartilage-derived stem cells*

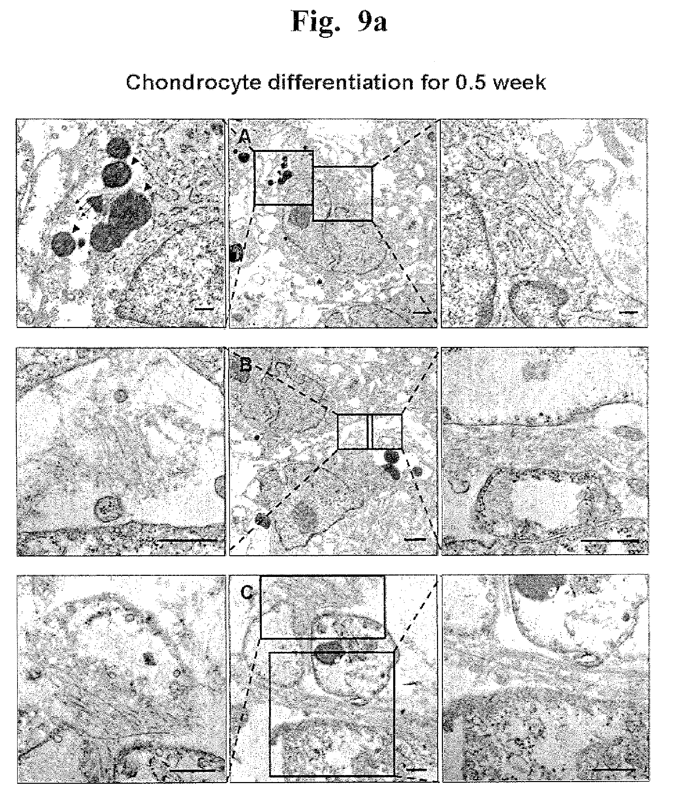

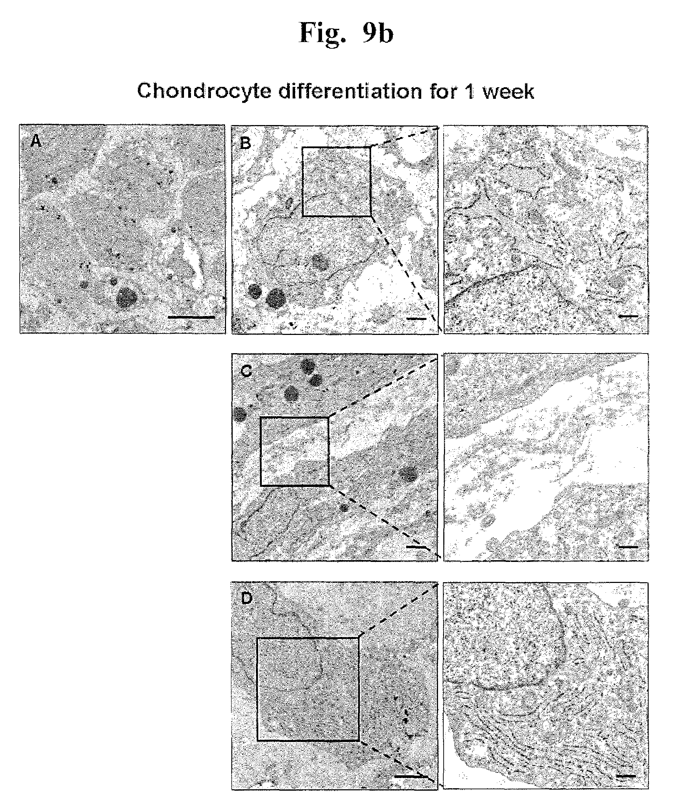

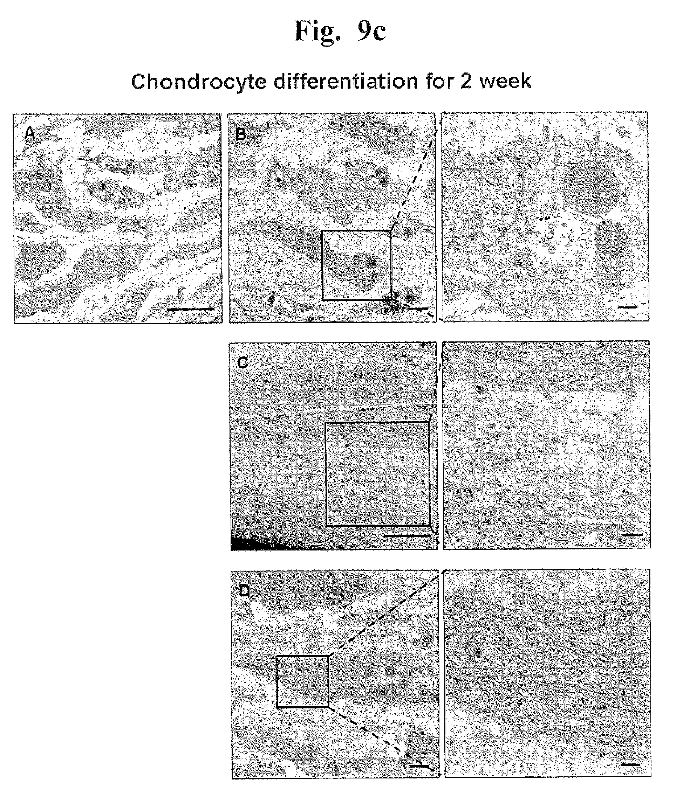

The cell pellets were observed under an electron microscope for morphological characterization of fine structure. The grown cell pellets were fixed in 2.5% glutaraldehyde-2% paraformaldehyde (4˚C, 0.1 M phosphate buffer, pH 7.4) for 2 hours and analyzed in the same manner as in Example 2. The cell pellets grown for 0.5 week showed irregular spherical shape on the whole. The nuclei were large, with spherical or irregular shape, and occupied a large portion of the cells. In the cytoplasm, a small number of small mitochondria were distributed near the nucleus. A number of well-developed secretory granules and free ribosomes were observed. Also, well-developed rough endoplasmic reticula were observed. Lipid droplets were observed as well (FIG. 9a, A). Between the cells, thin fiber bundles constituting the matrix of the cartilage were observed (FIG. 9a, B and C).

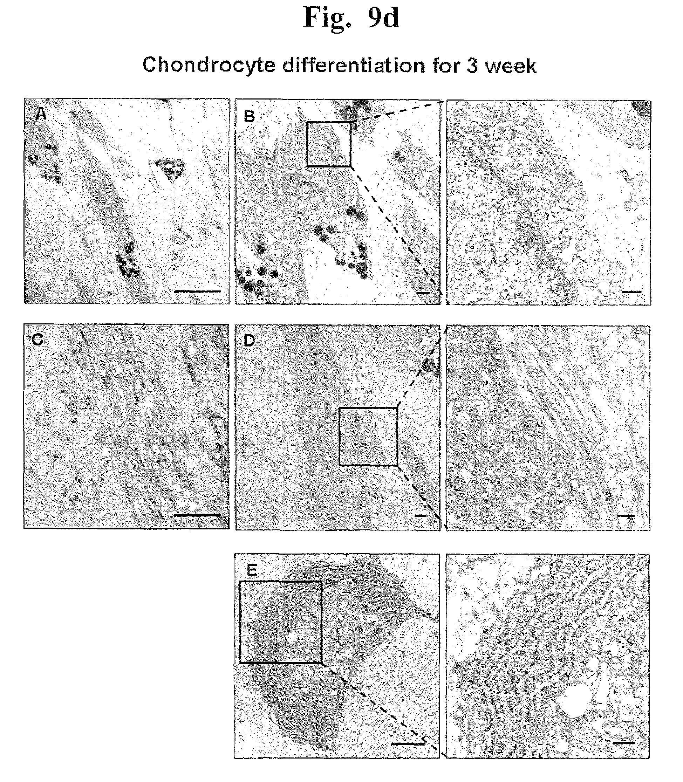

The cell pellets grown for 1 week showed oval shape, and the proportion of the nucleus in the cell was large (FIG. 9b, A). Free ribosomes and activated mitochondria and rough endoplasmic reticula near the nucleus were developed well in the cytoplasm. Lipid droplets were also observed (FIG. 9b, B). Between the cells, thin fiber bundles constituting the matrix of the cartilage were observed (FIG. 9b, C). Secretory vesicles were observed around the Golgi apparatuses (FIG. 9b, C and D).

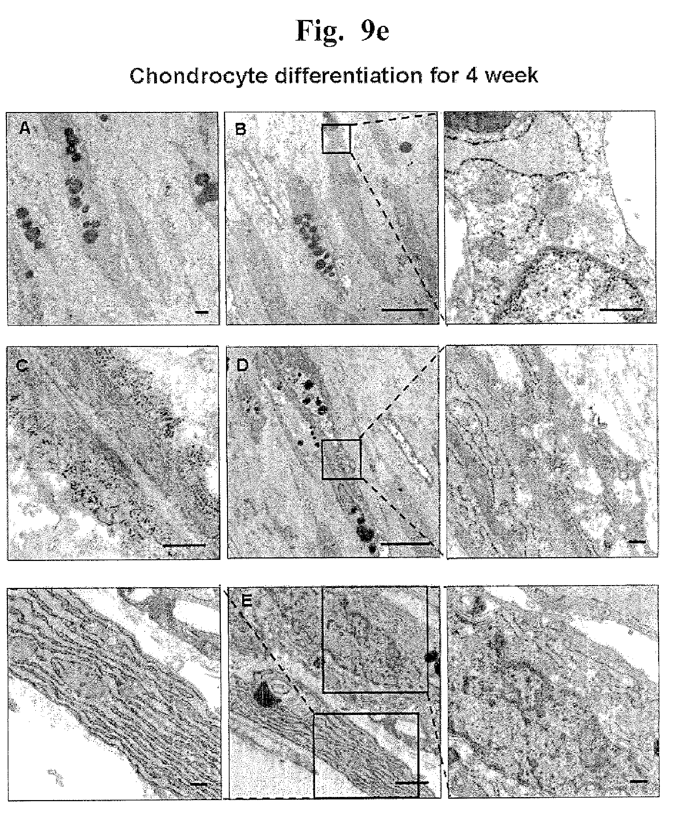

The cell pellets grown for 2, 3 and 4 weeks showed irregular, elongation shape on the whole. In the cytoplasm, activated mitochondria were distributed near the nucleus and a number of well-developed free ribosomes were observed. Also, the rough endoplasmic reticula were developed well. Besides, a number of lipid droplets were developed (FIG. 9c, A and B; FIG. 9d, A and B; FIG. 9e, A and B). Between the cells, thin fiber bundles constituting the matrix of the cartilage were observed (FIG. 9c, C and D; FIG. 9a, C, D and E).

*Analysis of stem cell marker genes and cell differentiation marker genes in cartilage -derived stem cells*

In order to investigate the characteristics of the monolayer-cultured cells isolated from the cartilage tissue and the cells cultured into pellets as stem cells and their differentiation into chondrocytes in gene level, the expression pattern of undifferentiated stem cell marker genes and cartilage differentiation-related marker genes was analyzed.

The total RNA of monolayer-cultured cells and pellet-cultured cells isolated from the cartilage tissue was isolated using the TRizol reagent (Invitrogen, USA). After homogenizing the pellet-cultured cells using a homogenizer, total RNA was isolated. After dissolving in RNase-free water, absorbance was measured at 260 nm (Bio-Rad, USA).

cDNA was synthesized using DNase I. After performing reverse transcription using the extracted total RNA (3 μg) as template and using DNase I (Roche Diagnostics, Germany), Super Script II reverse transcriptase (Invitrogen, USA), oligo d(T) (Invitrogen, USA) and dNTP (Invitrogen, US) at 42 ˚C for 60 minutes and at 72 ˚C for 15 minutes, the synthesized cDNA was kept at 4 ˚C.

PCR was performed using 25 μL of PCR premixture (2x Multiplex PCR PreMix; SolGent Co., Ltd., Korea), 1 μL of cDNA, and 2 μL of marker genes and forward & reverse primers (10 pmol) with the final volume of 50 μL, under the condition of denaturation at 95 ˚C for 45 seconds, annealing at 53-56˚C for 1 minute, and elongation at 72˚C for 2 minutes. 25-30 cycles were carried out in a T3 thermocycler (Biometra, Germany).

The primers were prepared based on the base sequence data from the US National Center for Biotechnology Information (NCBI, http-//www.ncbi.nlm.nih.gov) using the primer design software presented by Integrated DNA Technologies (http://www.idtdna.com). Primer sequences of the genes used in the PCR, product size, annealing temperature and number of PCR cycles are described in Table 6. The PCR product was electrophoresed on 2% agarose gel, stained with ethidium bromide (EtBr) and detected under UV. GAPDH was used as control.

The expression of the undifferentiated stem cells marker genes Oct3/4, Nanog and Rex-1 increased in the monolayer-cultured cells, indicating that the undifferentiated state was maintained. Among the major components of the cartilage matrix, type I, III and X collagens showed increased expression from the early stage of cartilage differentiation, i.e. 0.5 week of the pellet culturing. The expression of type II collagen increased in the middle stage of cartilage differentiation, i.e. 1 and 2 weeks of the pellet culturing, and decreased thereafter. The expression of type IX collagen increased strongly from the late stage of cartilage differentiation, i.e. 4 weeks of the pellet culturing. The expression of the cartilage matrix component aggrecan increased from the 1st week and decreased from the 3rd week. And, the expression of the genes Sox 5, Sox 6 and Sox 9 involved in the proliferation and differentiation control of chondrocytes increased strongly from 0.5 week and decreased after 2 weeks.

Table 6

| Primer sequence of marker genes and PCR condition | | | | | |
|---|---|---|---|---|---|
| Gene | | Primer Sequence(5'-3') | Product size | Annealing Temp. | Cycles |
| Oct3/4 | Forward | 5'- CGTGAAGCTGGAGAAGGAGAAGCTG -3' | 218 | 53 | 30 |
| | Reverse | 5'- CAAGGGCCGCAGCTTACATGTTC-3' | | | |
| Nanog | Forward | 5' -CAAAGGCAAACAACCCACTT -3' | 158 | 53 | 25 |
| | Reverse | 5' - TCTGCTGGAGGCTGAGGTAT -3' | | | |
| Rex1 | Forward | 5'-TGAAAGCCCACATCCTAACG -3' | 573 | 56 | 35 |
| | Reverse | 5'-CAAGCTATCCTCCTGCTTTGG -3' | | | |
| Sox5 | Forward | 5'-AGGGACTCCCGAGAGCTTAG -3' | 393 | 55 | 30 |
| | Reverse | 5'-GGGATCAGCTGMCAGGGTA -3' | | | |
| Sox6 | Forward | 5'-CTGCAACAGCAGCACAAAAT -3' | 294 | 55 | 30 |
| | Reverse | 5'-TAGGCCCTTTAGCCTTTGGT -3' | | | |
| Sox9 | Forward | 5'-GAACGCACATCAAGACGGAG -3' | 631 | 55 | 35 |
| | Reverse | 5'-TCTCGTTGATTTCGCTGCTC -3' | | | |
| Aggreca n | Forward | 5'-CCCGCTACGACGCCATCTG -3' | 427 | 55 | 35 |
| | Reverse | 5'-CGGGCGGTAGTGGAAGACG -3' | | | |
| Type collagen | Forward | 5'-CATCTCCCCTTCGTTTTTGA -3' | 598 | 53 | 30 |
| | Reverse | 5'-CTGTGGAGGAGGGTTTCAGA -3' | | | |
| Type □ collagen | Forward | 5'-ACATACCGGTAAGTGGGGCAAGAC -3' | 172 | 55 | 30 |
| | Reverse | 5'-GGATTGTGTTGTTTCTGGGTTC -3' | | | |

(continued)

| Gene | | Primer Sequence(5'-3') | Product size | Annealing Temp. | Cycles |
|---|---|---|---|---|---|
| | | Primer sequence of marker genes and PCR condition | | | |
| Type ☐ collagen | Forward | 5'-AAAGGGGAGCTGGCTACTTC -3' | 274 | 53 | 30 |
| | Reverse | 5'-GCGAGTAGGAGCAGTTGGAC -3' | | | |
| Type ☐ collagen | Forward | 5'-TGGCCAAGATGACTTACCAGGGTT -3' | 286 | 63 | 30 |
| | Reverse | 5'-TATGCCAACTTGCTCCTTCCCCAGA -3' | | | |
| Type ☐ collagen | Forward | 5'-CCCTTTTTGCTGCTAGTATCC -3' | 468 | 56 | 30 |
| | Reverse | 5'-CTGTTGTCCAGGTTTTCCTGGCAC -3' | | | |
| GAPDH | Forward | 5'-CCACAGTCCAGGCCATCACT -3' | 403 | 55 | 25 |
| | Reverse | 5'-GAGCTTGACAAAGTGGTCGT -3' | | | |

*Histochemical analysis of pellet-cultured cells*

Histochemical analysis was performed on the pellet-cultured cells. Through staining with safranin O, Alcian blue and trichrome, the expression pattern of the main cartilage matrix components of proteoglycan, glycosaminoglycan (GAG) and collagen was investigated.

The pellet-cultured cells were washed 3 times with DPBS and fixed in 10% neutral-buffered formalin (NBF) for 2 hours. The fixed cells were embedded in paraffin following a general tissue sample preparation procedure. After preparing 6-$\mu$m thick tissue sections using a microtome followed by deparaffinization and hydration, the sections were histochemically stained.

The nucleus and cytoplasm of the cell were stained by hematoxylin and eosin staining (Harris, 1900). After staining with hematoxylin solution for 3-5 minutes and washing, the sections were stained for 2 minutes in eosin solution and then washed, dehydrated, rinsed and embedded.

The portions containing proteoglycan, which is the main component of the cartilage and a viscous protein-polysaccharide complex, were stained with safranin O. After staining in Weigert's iron hematoxylin solution for 7 minutes and then washing, staining was carried out in 0.01% fast green solution for 3 minutes. After reaction in 1% acetic acid solution for 10-15 seconds, staining in 0.1% safranin O solution for 5 minutes was followed by washing, dehydration, rinsing and embedding.

The portions containing glycosaminoglycan, a major component of the cartilage, was stained with Alcian blue. After staining in Weigert's iron hematoxylin solution for 7 minutes and then washing, reaction was performed in 3% glacial acetic acid for 5 minutes. Then staining was performed in 1% Alcian blue solution for 30 minutes, which was followed by washing, dehydration, rinsing and embedding.

The portions containing collagen, a major component of the cartilage matrix, was stained with trichrome (Masson's trichrome staining). Reaction in Bouin solution at 56 ˚C for 1 hour and washing were followed by staining in Weigert's iron hematoxylin solution for 7 minutes and washing. Then, staining in Biebrich scarlet-acid fuchsin solution for 2 minutes and washing were followed by reaction in phosphomolybdic-phosphotungstic acid solution for 10-15 minutes and staining in aniline blue solution for 5 minutes. Finally, reaction in 0.5% glacial acetic acid solution for 5 minutes was followed by washing, dehydration, rinsing and embedding.

The analysis result revealed increased intensity of staining with increased pellet culture period and formation of cartilage lacuna.

**Example 4. Efficacy of cartilage stem cells for cell therapy**

*Test animals and management*

One hundred and twenty (120) 6-week-old male Sprague-Dawley rats (SLC, Japan) were used for test after accommodation for 7 days. All the test animals were kept in a polycarbonate cage, four or five each, and reared in a laboratory with temperature (20-25 °C) and humidity (40-45%) controlled. The light/dark cycle was 12 hours:12 hours, and feed (Samyang Corporation, Korea) and drinking water well provided freely. All the test animals had been fasted for at least 18 hours before sacrificing. They were treated according to "Guide for the Care and Use of Laboratory Animals by Institute of Laboratory Animal Resources, Commission on Life Science, National Research Council, USA on 1996, Washington DC". On day 7 after joint surgery, the rats were grouped into 7 groups as described in Table 4 based on joint thickness and body weight. 8 rats were selected from each group (56 in total) for test.

*Preparation and administration of cartilage stem cells and control drug (diclofenac sodium)*

The cells were suspended in CSBM at four different concentrations (high concentration: $9.6 \times 10^6$ cells/rat, intermediate high concentration: $4.8 \times 10^6$ cells/rat, intermediate low concentration: $2.4 \times 10^6$ cells/rat, low concentration: $1.2 \times 10^6$ cells/rat). One week after osteoarthritis inducing surgery (partial excision of the anterior cruciate ligament and the medial meniscus), the cells or the control drug were administered once into the articular capsule at a dosage of 1 mL/kg using CSBM. For the normal group and the osteoarthritis-induced control group, only the CSBM was administered into the articular capsule. As the control drug, 2 mg/kg diclofenac sodium (Sigma, MO, USA) dissolved in physiological saline was subcutaneously administered at the neck at a dose of 1 mL/kg, once a day for 84 days (Table 7).

Table 7

| Administration condition of cells or drug | |
|---|---|
| Test groups | Administration condition of cells or drug |
| Normal control group | The articular capsule was exposed via excision of muscles and 1 mL/kg of CSBM was administered without partial excision of the anterior cruciate ligament or the medial meniscus. |
| Osteoarthritis-induced control group | 1 mL/kg of CSBM was administered after joint surgery. |
| Control drug group | 2 mg/kg of diclofenac sodium was subcutaneously administered after joint surgery once a day for 84 days. |
| High-concentration cell group | Cells at high concentration were administered once into the articular capsule after joint surgery. |
| Intermediate high-concentration cell group | Cells at intermediate high concentration were administered once into the articular capsule after joint surgery. |
| Intermediate low-concentration cell group | Cells at intermediate low concentration were administered once into the articular capsule after joint surgery. |
| Low-concentration cell group | Cells at low concentration were administered once into the articular capsule after joint surgery. |

*Change in knee joint thickness*

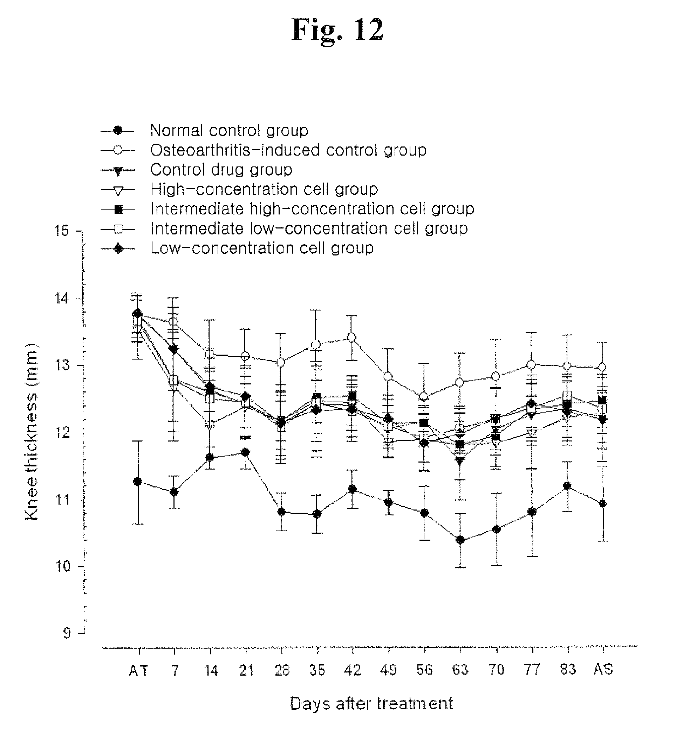

Osteoarthritis is a chronic inflammatory disease. Induced osteoarthritis leads to cartilage damage, causing edema of nearby joint and significant increase in joint thickness. The change in joint thickness is the most basic measure of evaluating induced osteoarthritis. A smaller increase in the joint thickness indicates less inducement of osteoarthritis.
The joint thickness of the rats in the control groups and test groups was measured using calipers. The osteoarthritis-induced control group showed significant increase in knee joint thickness of 18.66% as compared to the normal control group. The control drug group, the high-concentration cell group, the intermediate high-concentration cell group, the intermediate low-concentration cell group and the low-concentration cell group showed reduced knee joint thickness as compared to the osteoarthritis control group, with 5.79, 5.10, 3.82, 4.72 and 6.00%, respectively (FIG. 12).
All the cell administration groups and the control drug group showed significantly reduced knee joint thickness, indicating that the cartilage-derived stem cells significantly reduce edema induced by osteoarthritis.

*Change in maximum knee joint angle*

Induced osteoarthritis leads to fibrosis due to chronic inflammation and, as a result, the movement of the joint is greatly restricted. The maximum joint angle is used as the most basic index in evaluating the motility of the joint. A smaller angle indicates better motility (Rezende et al., 2006).

The administration groups of the drug and cell were sacrificed after 84 days. After taking out the right hind leg from the hip joint and removing nearby skin, connective tissues and muscles, the maximum knee joint angle was measured with the theoretical maximum as 0˚. All the measurement was performed by the same object in order to minimize error (Rezende et al., 2006).

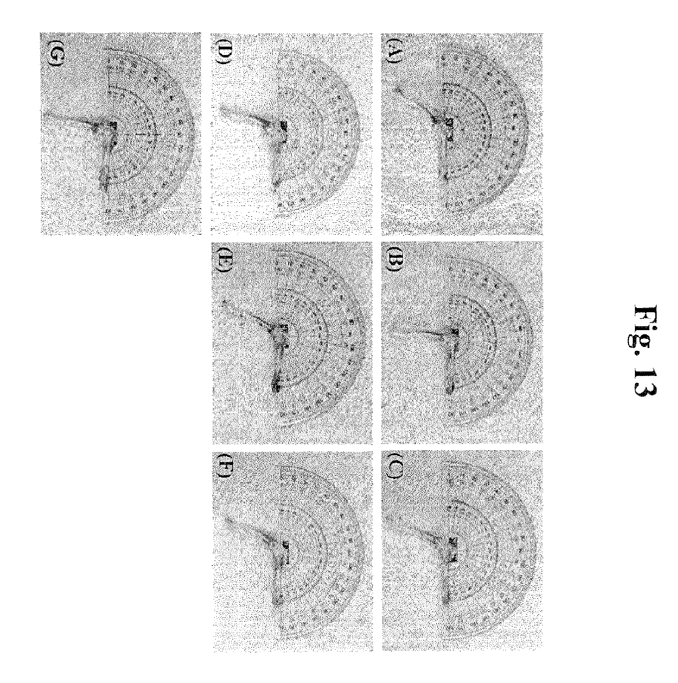

As a result, the osteoarthritis-induced control group (FIG. 13, B) showed significantly increased (159.39%) maximum knee joint angle as compared to the normal control group (FIG. 13, A). The control drug group (FIG. 13, C), the high-concentration cell group (FIG. 13, D), the intermediate high-concentration cell group (FIG. 13, E), the intermediate low-concentration cell group (FIG. 13, F) and the low-concentration cell group (FIG. 13, G) respectively showed knee joint angle decreased by 18.18, 21.72, 29.29, 16.16 and 15.99%, respectively, as compared to the normal control group. Thus, it was confirmed that the administration of the control drug or the cells effectively reduces maximum knee joint angle (Table 8).

Table 8

| Maximum knee joint angle | |
| --- | --- |
| Groups | Maximum knee joint angle (˚) |
| Normal control group | 28,63±3.20 |
| Osteoarthritis-induced control group | 74.25±4,13 |
| Control drug group | 60.75±6.78 |
| High-concentration cell group | 58.13±2.75 |
| Intermediate high-concentration cell group | 52.50±7.09 |
| Intermediate low-concentration cell group | 62.25±6.36 |
| Low-concentration cell group | 62.38±4.07 |

*Histopathological analysis of knee cartilage*

For histopathological observation and analysis of the cartilage Mankin score and cartilage thickness were measured (Table 9). Mankin score is frequently used to evaluate arthritis. A higher score is known to be related with higher inducement of osteoarthritis (Armstrong et al., log4; Rezende et al., 2006).

The articular capsule taken from the knee joint of each control group and test group was fixed for 5 days in 10% neutral-buffered formalin (NBF) and decalcified in decalcifying solution (24.4% formic acid, and 0.5 N sodium hydroxide). The decalcifying solution was replaced once a day for 5 days. After the decalcification, the median joint parts of the articular capsule were cut transversely and embedded in paraffin. After preparing 3-4 $\mu$m thick tissue sections, followed by deparaffinization and hydration, the cartilage was observed after hematoxylin & eosin (H&E) staining and safranin staining (Camplejohn and Allard, 1988; Kahveci et al., 2000; Tran et al., 2000).

Mankin score was measured by comparing damage to the knee joint tissue with that of the normal control group. Cartilage thickness of the tibia and the femur was measured by histomorphometry using an automatic image analyzer (DMI-300, DMI, Korea).

For the femur, the osteoarthritis-induced control group showed Mankin score increased by 1,216.67% as compared to the normal control group. The control drug group, the high-concentration cell group, the intermediate high-concentration cell group, the intermediate low-concentration cell group and the low-concentration cell group showed decrease by 27.85, 69.62, 73.42, 50.63 and 51.90%, respectively, as compared to the osteoarthritis-induced control group (Table 10). And, the osteoarthritis-induced control group showed cartilage thickness increased by 46-78% as compared to the normal control group. The control drug group, the high-concentration cell group, the intermediate high-concentration cell group, the intermediate low-concentration cell group and the low-concentration cell group showed increase by 35.11, 130.49, 155.18, 107.53 and 109.59%, respectively, as compared to the osteoarthritis-induced control group (Table 12).

For the tibia, the osteoarthritis-induced control group showed Mankin score increased by 2166.67% as compared to the normal control group. The control drug group, the high-concentration cell group, the intermediate high-concentration cell group, the intermediate low-concentration cell group and the low-concentration cell group showed decrease by 20.59, 39.71, 44.12, 42.65 and 36.76%, %, respectively, as compared to the osteoarthritis-induced control group (Table 11). And, the osteoarthritis-induced control group showed cartilage thickness increased by 61.99% compared to the normal control group. The control drug group, the high-concentration cell group, the intermediate high-concentration cell group, the intermediate low-concentration cell group and the low-concentration cell group showed increase by 68.81, 104.12, 137.91, 96.84 and 116.46%, respectively, as compared to the osteoarthritis-induced control group (Table 12).

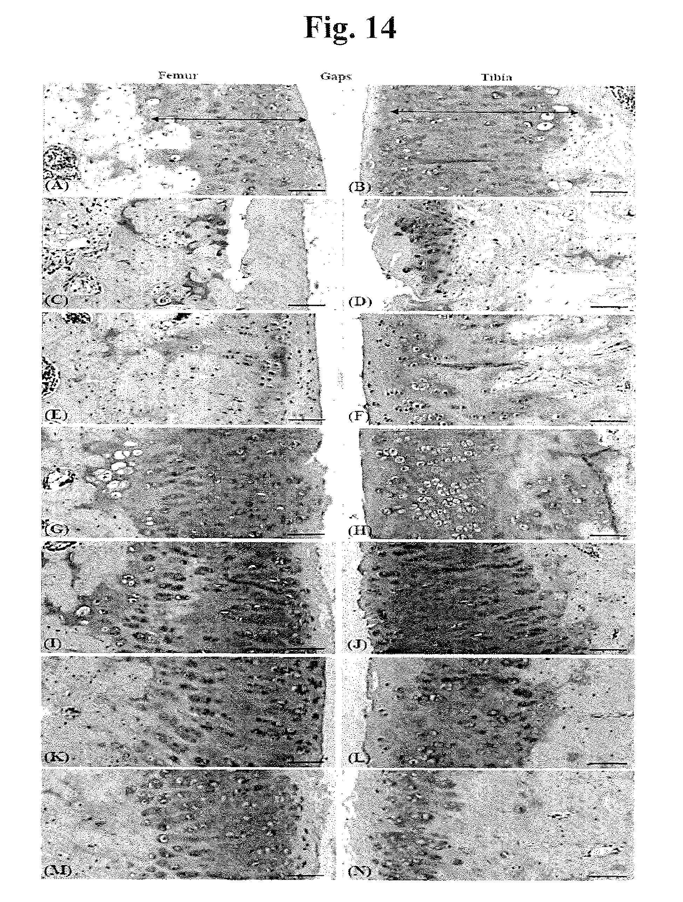

To conclude, the osteoarthritis group showed increased Mankin score for the tibia and the femur and significantly reduced cartilage thickness (Moore et al., 2005). In contrast, the control drug group and the cell groups showed decreased Mankin score and cartilage thickness recovered comparable to that of the normal control group (Table 7, 8, 9, FIG. 14).

Table 9

| Mankin score (Max = 12) | |
|---|---|
| Surface | 0 = normal<br>1 = irregular<br>2 = fibrillation/vacuoles<br>3 = blisters/erosion |
| Hypocellularity | 0 = normal<br>1 = small decrease in chondrocytes<br>2 = large decrease in chondrocytes<br>3 = no cells |
| Clones | 0 = normal<br>1 = occasional duos<br>2 = duos/trios<br>3 = multiple nested cells |
| Stain intensity for safranin 0 | 0 = normal<br>1 = small decrease in color<br>2 = large decrease in color<br>3 = no color |

Mankin score is given by the sum.

Table 10

| Mankin score of the femur | | | | | |
|---|---|---|---|---|---|
| Groups | Surface | Hypocellularity | Clones | Safranin O | Sum[1] |
| Normal control group | 0.26±0.46 | 0.25±0.46 | 0.00±0.00 | 0.25±0.46 | 0-75±0.89 |
| Osteoarthritis-induced control group | 2.75±0.46 | 2.38±0.52 | 2.25±1.16 | 2.50±0.53 | 9.88±1.55 |
| Control drug group | 2.13±0.35 | 1.38±0.52 | 2.38±0.92 | 1.25:1:0.46 | 7.13±1.36 |
| High-concentration cell group | 1.63±0.74 | 0.63±0.74 | 0.63±0.74 | 0.13±0.35 | 3.00±2.20 |
| Intermediate high-concentration cell group | 1.63±0.74 | 0.25±0.46 | 0.63±0.74 | 0.13±0.35 | 2.63±1.92 |
| Intermediate low-concentration cell group | 2.00±0.93 | 1.00±0.53 | 1.75-±0.71 | 0.13±0.35 | 4.88±1.13 |
| Low-concentration cell group | 1.88±0.35 | 0.88±0.83 | 150±1.07 | 0.50±0.76 | 4.75±1.39 |
| 1) Mankin score (Max =12) | | | | | |

Table 11

| Mankin score of the tibia | | | | | |
|---|---|---|---|---|---|
| Groups | Surface | Hypocellularity | Clones | Safranin 0 | Sum[1] |
| Normal control group | 0.25±0.45 | 0.00±0.00 | 0.00±0.00 | 0.13±0.35 | 0.38±0.74 |
| Osteoarthritis-induced control group | 2.63±0.52 | 2.00±0.53 | 2.38±0.52 | 1.50±0.93 | 8.50±1.93 |
| Control drug group | 2.00±0.53 | 1.63±0.52 | 1.75±0.71 | 1.38+0.74 | 6.75+1.16 |
| High-concentration cell group | 2,00±0.93 | 1.13±0.83 | 1.38±1.19 | 0.63±0.92 | 5.13±3.64 |
| Intermediate high-concentration cell group | 1.88±0.64 | 0.88±0.83 | 1.50±1.07 | 0.50±0.76 | 4.75±2.76 |
| Intermediate low-concentration cell group | 2.13±0.35 | 1.13±0.64 | 1.25±0.46 | 0.38±0.52 | 4.88±1.55 |
| Low-concentration cell group | 1.88±0.64 | 0.75±0.71 | 2.00±1.20 | 0.75±0.89 | 5.38±2.88 |
| 1) Mankin score (Max = 12) | | | | | |

Table 12

| Knee cartilage thickness | | |
|---|---|---|
| Groups | Knee cartilage thickness | |
| | Femur | Tibia |
| Normal control group | 569.43±95.77 | 780.16±145.83 |
| Osteoarthritis-induced control group | 303.07±91.40 | 296.51±73.69 |
| Control drug group | 409.48±69.80 | 500.53±196.69 |
| High-concentration cell group | 698.57±158.23 | 605.23-±200.40 |
| Intermediate high-concentration cell group | 773.39±149.62 | 705.42±218.46 |
| Intermediate low-concentration cell group | 628.96±161.61 | 585.66±177.11 |
| Low-concentration cell group | 635.21±212.37 | 641.67±202.63 |

*Growth of chondrocytes in knee cartilage*

Chondrocytes growing in the articular capsule were labeled with BrdU in order to investigate their growth due to the administration of the cartilage-derived stem cells. Since the cells labeled with BrdU are those grown after the injection of BrdU, the growth of chondrocytes can be analyzed.

5-Bromo-2'-deoxyuridine (50 mg/kg, BrdU; MP Biomedicals, OH, USA) diluted in physiological saline to a concentration of 2 mL/kg was intraabdominally injected once 72 hours before the sacrificing of the rats of the control groups and test groups. Then, immunohistochemical staining was performed using BrdU antibody (Hwang et al., 2000; Moore et al., 2005; Ganey et al., 2003; Ito et al., 1998; Tang et al., 2007).

The expression pattern of BrdU was investigated using the Vector Elite ABC kit (Vector Lab. Inc., CA, USA). After washing the tissue with DPBS, reaction was performed in 3% hydrogen peroxide ($H_2O_2$) solution for 30 minutes at room temperature. After washing with DPBS, reaction was performed for 30 minutes using normal horse serum (1: 100, Vector Lab. Inc., CA, USA) in order to prevent nonspecific reaction that might occur during the staining. After reaction with the primary antibody anti-mouse BrdU (1:100, Abcam, England) at 4 °C for 18 hours followed by washing with DPBS, reaction was performed with biotinylated secondary antibody at room temperature for 30 minutes. After washing excess secondary antibody with DPBS, reaction was performed at room temperature for 1 hour in ABC solution (diluted 1:50) included in the Vector Elite ABC kit. After washing again with DPBS and inducing color reaction using a peroxidase substrate kit (Vector Lab. Inc., CA. USA), counterstaining with Mayer's hematoxylin solution was followed by washing, dehydration, rinsing and embedding. To identify the specificity of staining, the negative control group was stained in the same way but without the primary antibody and observed under an optical microscope (Nikon, Japan). The number of cells responding to BrdU antibody by 10% or more was counted. From a total of 100 chondrocytes on the articular surface of the tibia and the femur, the percentage of BrdU-positive cells was measured using an automatic image analyzer.

As a result, the number of BrdU-positive cells on the articular surface of the femur decreased by 82.69% in the osteoarthritis-induced control group as compared to the normal control group. The control drug group, the high-concentration cell group, the intermediate high-concentration cell group, the intermediate low-concentration cell group and the low-concentration cell group showed increase by 9.52%, 298.41%, 400.00%, 233.33% and 206-35%, respectively, as compared to the osteoarthritis-induced control group (Table 13).

The number of BrdU-positive cells on the articular surface of the tibia decreased by 80.43% in the osteoarthritis-induced control group as compared to the normal control group. The control drug group, the high-concentration cell group, the intermediate high-concentration cell group, the intermediate low-concentration cell group and the low-concentration cell group showed increase by 1.56%, 265,63%, 401,56%, 314.06%, 314.06% and 209,38%, respectively, as compared to the osteoarthritis-induced control group (Table 13).

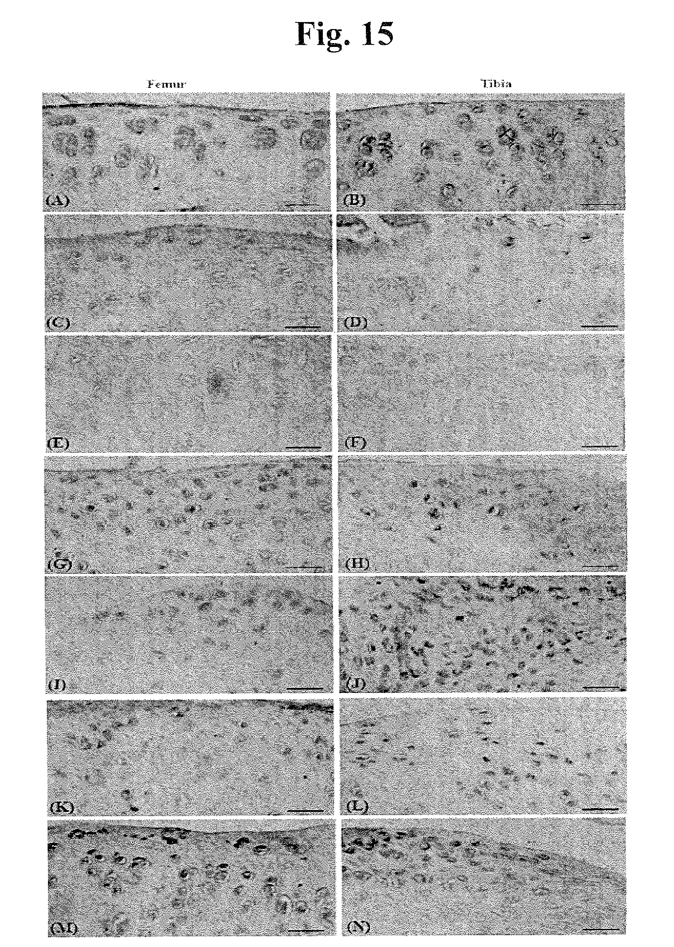

To conclude, the osteoarthritis-induced control group showed significant decrease in the number of BrdU-positive cells as compared to the normal control group. Whereas the control drug group showed a similar number of BrdU-positive cells to that of the osteoarthritis-induced control group, all the cartilage-derived stem cell groups showed increased number of BrdU-positive cells as compared to that of the osteoarthritis-induced control group. Also, the cartilage stem cell groups showed recovery of the cartilage damage to a level comparable to the normal control group (FIG. 15).

Table 13

| Number of BrdU-positive cells in knee cartilage | | |
|---|---|---|
| Groups | Number of BrdU-positive cells /100 chondrocytes | |
| | Femur | Tibia |
| Normal control group | 45.50±9.49 | 40.88±8.43 |
| Osteoarthritis-induced control group | 7.88±3.56 | 8.00±4.24 |
| Control drug group | 8.63+4.03 | 8.13±3.52 |
| High-concentration cell group | 31.38±8.37 | 29.25±8.84 |
| Intermediate high-concentration cell group | 39.38+11.70 | 40.133±10.18 |
| Intermediate low-concentration cell group | 26.25±3.37 | 33.13±8.32 |
| Low-concentration cell group | 24.13±9.40 | 24.75±11.32 |

*Analysis of cellular distribution by immunohistochemical staining*

The expression pattern of human-specific mitochondria and cartilage marker protein human-specific type II collagen was analyzed immunohistochemical staining in order to investigate the distribution of the cells administered to the test groups.

The articular capsule from the knee joint of the test group rats was taken out, fixed in 10% neutral-buffered formalin for 5 days, and decalcified using decalcifying solution. The decalcifying solution was replaced once a day for 5 days. After the decalcification, the median joint parts of the articular capsule were cut transversely and embedded in paraffin. After preparing 3-4 $\mu$m thick tissue sections, followed by deparaffinization, hydration and washing with distilled water, the sections were boiled for 5 minutes in sodium citrate solution (10 mM, pH 6.0) containing 0.05% Tween 20, followed by immunohistochemical staining.

The tissue sections were washed with DPBS and reacted with 3% bovine serum albumin (BSA; Sigma, USA) for 45 minutes to prevent nonspecific reaction that might occur during the staining. Following the blocking and washing 3 times with DPBS, the tissue sections were reacted respectively with the primary antibodies human-specific anti-mouse mitochondria (1:50, Chemicon, USA) and human-specific anti-rabbit type **II** collagen (1:50, Chemicon, USA) at 4 °C for 14-16 hours. Then, after washing 3 times with DPBS, reaction was performed with FITC-conjugated anti-rabbit (1: 500, Invitrogen, USA) and Cy3-conjugated anti-goat (1:500, Invitrogen, USA) secondary antibodies for 3 hours. Then, after reacting with the nuclear marker DAPI (1:1000, Sigma, USA) for 10 minutes and washing 3 times with DPBS, protein expression was analyzed by confocal microscopy (LSM 510, Zeiss, Germany).

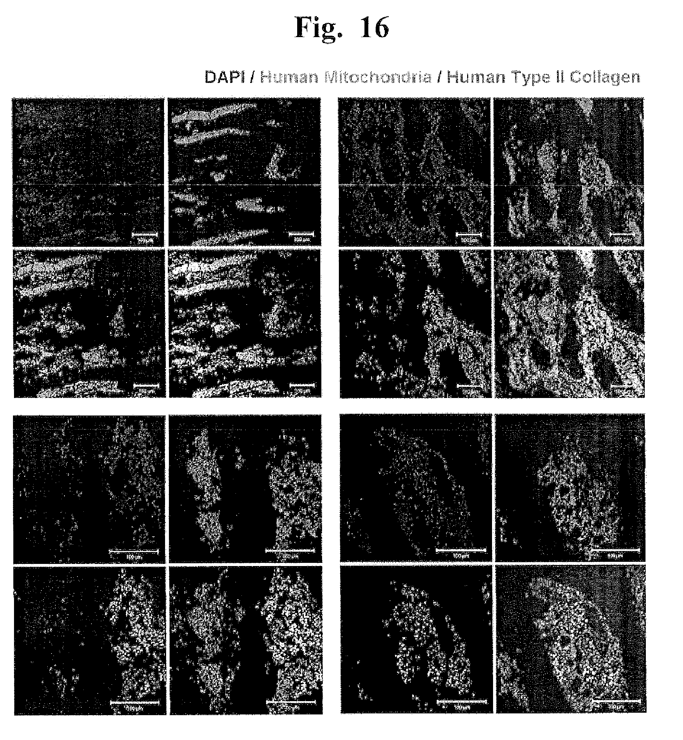

As a result, human-specific mitochondria and the portions exhibiting strong expression of the protein human-specific type II collagen were identified in the tissue sections of the test groups (FIG. 16).

*Equations for evaluation*

Percent change between the normal control group and the osteoarthritis-induced control group was calculated by Equation 1 in order to evaluate the degree of inducement of osteoarthritis. Also, percent change between the cell administration group and the osteoarthritis-induced control group was calculated by Equation 2 in order to more clearly represent the efficacy of the administered cells.

Equation 1

Percent change (%) between the normal control group and the osteoarthritis-induced control group

= [(Value of the osteoarthritis-induced control group - Value of the normal control group) / Value of the normal control group] x 100

Equation 2

Percent change (%) between the cell administration group and the osteoarthritis-induced control group

= [(Value of the cell administration group - Value of the osteoarthritis-induced control group) / Value of the osteoarthritis-induced control group] x 100

While the present disclosure has been described with respect to the specific embodiments, it will be apparent to those skilled in the art that various changes and modifications may be made without departing from the spirit and scope of the disclosure as defined in the following claims.

**References**

1. Armstrong S, Read R, Ghosh P. The effects of intra articular hyaluronan on cartilage and subchondral bone changes in an ovine model of early osteoarthritis. J Rheumatol. 1994; 21: 680-8,
2. Camplejohn KL, Allard SA. Limitations of safranin 'O' staining in proteoglycan-depleted cartilage demonstrated with monoclonal antibodies. Histochemistry. 1988; 89: 185-8.
3. Kahveci Z, Minbay FZ, Cavusoglu L. Safranin O staining using a microwave oven. Biotech Histochem. 2000; 75: 264-8.
4. Moore EE, Bendele AM, Thompson DL, Littau A, Waggie KS, Reardon B, Ellsworth JL. Fibroblast growth factor-18 stimulates chondrogenesis and cartilage repair in a rat model of injury-induced osteoarthritis. Osteoarthritis Cartilage. 2005; 13:623-31.
5. Rezende MU, Gurgel HM, Vilaca Junior PR, Kuroba RK, Lopes AS, Phillipi RZ, Hernandez AJ. Diacerhein versus glucosamine in a rat model of osteoarthritis. Clinics. 2006; 61: 461-6.
6. Tran D, Golick M, Rabinovitz H, Rivlin D, Elgart G, Nordlow B. Hematoxylin and safranin O staining of frozen sections. 2000; Dermatol Surg. 26: 197-9.

**Claims**

1. A pharmaceutical composition for treating bone disease, comprising:

   (a) (i) a therapeutically effective amount of cartilage stem cells being immunologically positive for at least one surface antigen selected from a group consisting of CD29, CD44, CD49C, CD73 and CD105, and (ii) being immunologically negative for the surface antigen CD34 or CD45, and (iii) expressing at least one undifferentiated stem cell marker protein selected from a group consisting of Oct3/4, Nanog, Rex-1, Sox 2, Sox 5, Sox 6 and Sox 9 in the cytoplasm; and
   (b) a pharmaceutically acceptable excipient.

2. The composition according to claim 1, wherein the population doubling time of the cartilage stem cells is 15-30 hours.

3. The composition according to claim 1, wherein the cartilage stem cells do not express the histocompatibility antigen HLA-DR (MHC class II).

4. The composition according to claim 1, wherein the cartilage stem cells are autologous cells or exogenous cells.

5. The composition according to claim 1, wherein the cartilage stem cells express the cytoskeleton marker protein vimentin or the intercellular cell adhesion protein CD54 (intercellular cell adhesion molecule-1: ICAM-1) in the cytoplasm.

6. The composition according to claim 1, wherein the cartilage stem cells express the intercellular adhesion protein CD44 (homing cell adhesion molecule: HCAM) in the cell membrane.

7. The composition according to claim 1, wherein the cartilage stem cells are the cells deposited under the accession numbers KCTC 11397BP "CBAC266", KCTC 11617BP "CBAC237", KCTC 11618BP "CBAC240" or KCTC 11619BP "CBAC1014".

8. The composition according to claim 1, wherein the cartilage stem cells are derived from human cartilage tissue.

9. The composition according to claim 1, wherein the cartilage stem cells exhibit an anti-inflammatory activity.

10. The composition according to claim 1, wherein the cartilage stem cells exhibit an activity of promoting growth of chondrocytes.

11. The composition according to claim 1, wherein the bone disease is caused by pathological or physical bone damage.

12. A pharmaceutical composition for countering inflammation, comprising:

   (a) (i) a therapeutically effective amount of cartilage stem cells being immunologically positive for at least one surface antigen selected from a group consisting of CD29, CD44, CD49C, CD73 and CD105, and (ii) being immunologically negative for the surface antigen CD34 or CD45, and (iii) expressing at least one undifferentiated stem cell marker protein selected from a group consisting of Oct3/4, Nanog, Rex-1, Sox 2, Sox 5, Sox 6 and Sox 9 in the cytoplasm; and
   (b) a pharmaceutically acceptable excipient.

13. The composition according to claim 12, wherein the population doubling time of the cartilage stem cells is 15-30 hours.

14. The composition according to claim 12, wherein the cartilage stem cells do not express the histocompatibility antigen HLA-DR (MHC class II).

15. The composition according to claim 12, wherein the cartilage stem cells are autologous cells or exogenous cells.

16. The composition according to claim 12, wherein the cartilage stem cells express the cytoskeleton marker protein vimentin or the intercellular cell adhesion protein CD54 (intercellular cell adhesion molecule-1: ICAM-1) in the cytoplasm.

17. The composition according to claim 12, wherein the cartilage stem cells express the intercellular adhesion protein CD44 (homing cell adhesion molecule: HCAM) in the cell membrane.

18. The composition according to claim 12, wherein the cartilage stem cells are the cells deposited under the accession numbers KCTC 11397BP "CBAC266", KCTC 11617BP "CBAC237", KCTC 11618BP "CBAC240" or KCTC 11619BP "CBAC1014".

19. The composition according to claim 12, wherein the cartilage stem cells are derived from human cartilage tissue.

20. The composition according to claim 12, wherein the composition is a composition for intraarticular administration, and the composition suppresses inflammatory response in the joint.

# Fig. 1

# Fig. 1b

# Fig. 1c

# Fig. 1d

# Fig. 2a

**Fig. 2b**

# Fig. 2c

# Fig. 2d

# Fig. 3a

# Fig. 3b

# Fig. 3c

EP 2 377 542 A2

Fig. 3d

31

# Fig. 4a

# Fig. 4b

# Fig. 4c

# Fig. 4d

# Fig. 5

# Fig. 6

# Fig. 7

# Fig. 8

# Fig. 9a

## Chondrocyte differentiation for 0.5 week

# Fig. 9b

## Chondrocyte differentiation for 1 week

# Fig. 9c

## Chondrocyte differentiation for 2 week

# Fig. 9d

## Chondrocyte differentiation for 3 week

# Fig. 9e

## Chondrocyte differentiation for 4 week

# Fig. 10

# Fig. 11

H & E stain

Masson trichrome stain

Alcian blue stain

Safranin O stain

# Fig. 12

# Fig. 13

# Fig. 14

# Fig. 15

Femur                                    Tibia

# Fig. 16

DAPI / Human Mitochondria / Human Type II Collagen

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6835377 B

### Non-patent literature cited in the description

- **Majumdar M. K. et al.** *J. Cell. Physiol.,* 2000, vol. 185, 98-106
- **Gang E. J. et al.** *Biochem. Biophys. Res. Commun.,* 2004, vol. 321, 102-108
- **Fickert S. et al.** *Osteoarthritis Cartilage,* 2003, vol. 11, 790-800
- **Eagle, H.** *Science,* 1959, vol. 130, 432
- **Stanner, C.P. et al.** *Nat. New Biol.,* 1971, vol. 230, 52
- **Iscove, N. et al.** *J. Exp. Med.,* 1978, vol. 147, 923
- **Morgan et al.** *Proc. Soc. Exp. Bio. Med.,* 1950, vol. 73, 1
- **Moore et al.** *J. Amer. Med. Assoc.,* 1967, vol. 199, 519
- **Ham.** *Proc. Natl. Acad. Sci. USA,* 1965, vol. 53, 288
- **Ham, R.G.** *Exp. Cell Res.,* 1963, vol. 29, 515
- **Dulbecco, R. et al.** *Virology,* 1959, vol. 8, 396
- **Barnes, D. et al.** *Anal. Biochem.,* 1980, vol. 102, 255
- **Waymouth, C.** *J. Natl. Cancer Inst.,* 1959, vol. 22, 1003
- **McCoy, T.A. et al.** *Proc. Soc. Exp. Biol. Med.,* 1959, vol. 100, 115
- **Ham, R.G. et al.** *In Vitro,* 1978, vol. 14, 11
- Remington's Pharmaceutical Sciences. 1995

- Guide for the Care and Use of Laboratory Animals by Institute of Laboratory Animal Resources, Commission on Life Science. National Research Council, 1996
- **Armstrong S ; Read R ; Ghosh P.** The effects of intra articular hyaluronan on cartilage and subchondral bone changes in an ovine model of early osteoarthritis. *J Rheumatol.,* 1994, vol. 21, 680-8
- **Camplejohn KL ; Allard SA.** Limitations of safranin 'O' staining in proteoglycan-depleted cartilage demonstrated with monoclonal antibodies. *Histochemistry,* 1988, vol. 89, 185-8
- **Kahveci Z ; Minbay FZ ; Cavusoglu L.** Safranin O staining using a microwave oven. *Biotech Histochem.,* 2000, vol. 75, 264-8
- **Moore EE ; Bendele AM ; Thompson DL ; Littau A ; Waggie KS ; Reardon B ; Ellsworth JL.** Fibroblast growth factor-18 stimulates chondrogenesis and cartilage repair in a rat model of injury-induced osteoarthritis. *Osteoarthritis Cartilage,* 2005, vol. 13, 623-31
- **Rezende MU ; Gurgel HM ; Vilaca Junior PR ; Kuroba RK ; Lopes AS ; Phillipi RZ ; Hernandez AJ.** Diacerhein versus glucosamine in a rat model of osteoarthritis. *Clinics,* 2006, vol. 61, 461-6
- **Tran D ; Golick M ; Rabinovitz H ; Rivlin D ; Elgart G ; Nordlow B.** Hematoxylin and safranin O staining of frozen sections. *Dermatol Surg.,* 2000, vol. 26, 197-9